# EUROPEAN PATENT SPECIFICATION

(11) **EP 1 485 714 B1**
(45) Date of publication and mention of the grant of the patent: **03.12.2008**
(21) Application number: 03708361.5
(22) Date of filing: 20.03.2003
(51) Int. Cl.: G01N 33/50

(54) **METHODS FOR THE DETECTION OF PRION PROTEINS**
VERFAHREN FÜR DEN NACHWEIS VON PRIONPROTEINEN
PROCEDE POUR LA DETECTON DE PRIONS

(30) Priority: 20.03.2002 GB 0206585
(43) Date of publication of application: 15.12.2004
(73) Proprietor: D-Gen Limited, London W2 1NY (GB)
(72) Inventor: ENARI, Masato National Cancer Centre Res. Inst., Tokyo 104-0045 (JP); KLOEHN, Peter MRC Prion Unit, Queens Square London WC1N 3BG (GB); FLECHSIG, Eckhard Inst. Virology & Immunbiology, D-97078 Wuerzburg (DE); STOLTZE, Lars Dept. of Cellular Neurology, Otfried-Müller-Str. 27 72076 Tübingen (DE); WEISSMANN, Charles MRC Prion Unit, Queens Square London WC1N 3BG (GB)
(74) Representative: Richards, William John
(86) International application number: PCT/GB2003/001313
(87) International publication number: WO 2003/081249

(56) References cited:
- BIRKETT CHRISTOPHER R ET AL: "Scrapie strains maintain biological phenotypes on propagation in a cell line in culture." EMBO (EUROPEAN MOLECULAR BIOLOGY ORGANIZATION) JOURNAL, vol. 20, no. 13, 2 July 2001 (2001-07-02), pages 3351-3358, XP002254520 ISSN: 0261-4189
- ENARI M FLECHSIG E WEISSMANN C: "Scrapie prion protein accumulation by scrapie-infected neuroblastoma cells abrogated by exposure to a prion protein antibody" PROCEEDINGS OF THE NATIONAL ACADEMY OF SCIENCES OF USA, NATIONAL ACADEMY OF SCIENCE. WASHINGTON, US, vol. 98, no. 16, 31 July 2001 (2001-07-31), pages 9295-9299, XP002959455 ISSN: 0027-8424

## Description

### Field of the Invention

The present invention relates to a cell-culture-based (i.e. *in vitro*) assay for prions. In particular, the present invention relates to a quantitative *in vitro* assay for prions. The invention further relates to a cell line useful in the *in vitro* assay of prions.

### Background

During the course of prion disease, a largely protease-resistant, aggregated form of PrP, designated PrP^{Sc}, accumulates, mainly in brain. It is considered to be the main or only constituent of the prion (McKinley et al., 1991; Oesch et al., 1985). Because no differences in primary sequence were found between PrP^{C} and PrP^{Sc} (Stahl et al., 1993), the two species are believed to differ only in their conformation.

Although the presence of protease-resistant PrP in brain or lymphoreticular organs is a recognised, characteristic and specific feature of prion disease, it does not necessarily correlate with the level of infectious agent. Infectivity has hitherto been quantified by injecting the sample intracerebrally into an indicator mouse and determining the time to appearance of definitive clinical symptoms (incubation time method (Prusiner et al., 1982)) or by injecting a series of dilutions of the sample and determining at which dilution 50% of the mice acquire scrapie (endpoint method (Reed and Muench, 1938)).

A few cell lines, mostly derived from neural tissue, such as the N2a, GT1 and PC12 lines (Butler et al., 1988; Markovits et al., 1981; Race et al., 1987; Schätzl et al., 1997), but also an epithelial rabbit cell line (Vilette et al., 2001) and a mesodermal cell line (SMB cell line) (Birkette et al., 2001), can be - infected by prions, as evidenced by the accumulation of PrP^{Sc} and/or infectivity after multiple passages. Only a small fraction of an N2a or GT1 cell population is readily infected by the mouse-adapted Rocky Mountain Laboratory (RML) scrapie strain, but from N2a populations sublines have been established which are susceptible to infection (Bosque and Prusiner, 2000; Nishida *et al.,* 2000, Enari, 2001). N2a cells, and in particular selected sublines, are susceptible to prion infection, as evidenced by the accumulation of PrP^{Sc} and propagation of infectivity, however the process has been considered too inefficient to be used as a sensitive and quantitative assay. Bosque and Prusiner assessed the suitability of their N2a sublines for an *in vitro* assay by exposing them to increasing dilutions of scrapie-infected mouse brain homogenate and determining the dilution at which a blotting assay for PrP^{Sc} became negative. They reported that this cell culture assay was several orders of magnitude less sensitive than the *in vivo* mouse assay (Bosque and Prusiner, 2000). Moreover, Bosque and Prusiner (2000) evaluate "infection" by blackening of the film following the PrPSc blotting assay, but we disclose quantification by counting of individual colonies, which has the advantages explained herein.

Although the presence of protease-resistant PrP in brain or lymphoreticular organs is a recognised, characteristic feature of prion disease, it does not provide a measure for the level of infectious agent (Manuelidis et al., 1987; Sakaguchi et al., 1993).

In the prior art, infectivity has been quantified by introducing a sample into an indicator mouse, most commonly by intracerebral injection, and determining the time to appearance of definitive clinical scrapie symptoms (incubation time method, (Prusiner et al., 1982)). This method involves the upkeep of laboratory animals which is expensive and labour intensive. The assays take months to complete due to the time taken to develop and display clinical symptoms, even at relatively high infective doses.

Another prior art method involves injecting a series of dilutions of the sample into indicator mice and determining at which dilution 50% of the mice acquire scrapie (endpoint method, (Reed and Muench, 1938)). This method again requires large numbers of test animals, and incurs high maintenance and labour costs. Assessing the symptoms of scrapie is a time consuming process which can be open to subjectivity.

Although it has been observed that N2a cells and particular selected sublines can be infected with prions, prior art cell lines and methods have been considered too inefficient to be used as a sensitive and/or quantitative assay (Bosque and Prusiner, 2000). Prior art cell lines have not provided sufficient sensitivity for useful *in vitro* assays to be conducted, nor have they used "colony counting" techniques.

The present invention seeks to overcome problem(s) associated with the prior art.

### Summary of the Invention

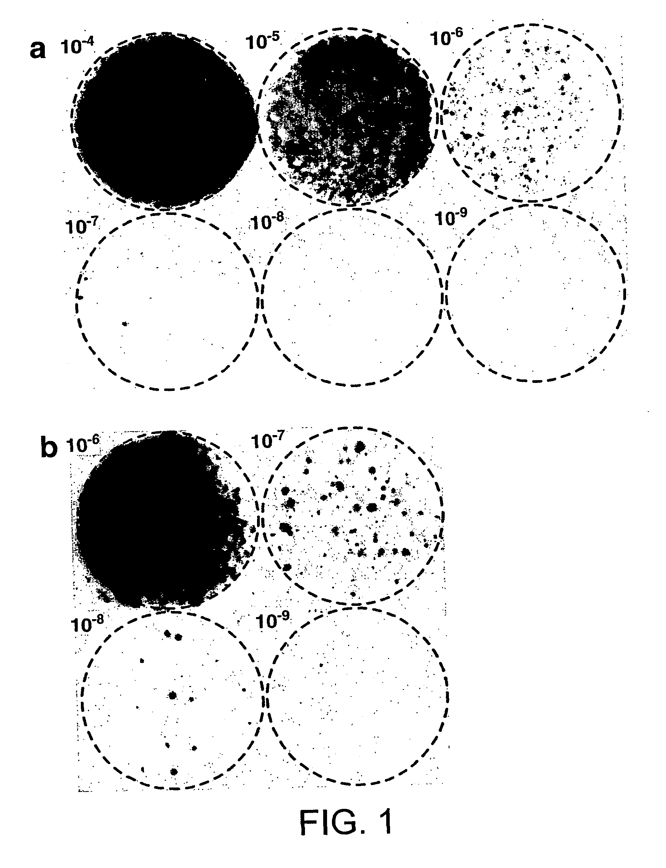

We have isolated a N2a subline, N2a/Gary, which is highly susceptible to prions such as RML scrapie prions. This N2a/Gary cell line may occasionally be referred to as N2aPD88 or N2aPD112 or even N2aPD. Non-susceptible N2a cell lines may occasionally be referred to as NN2a or N2aR33, or even N2aATCC. We found that monolayers of infected N2a cells grown on coverslips, when blotted onto membranes, and assayed for PrP^{Sc}, gave a speckled pattern, suggesting that micro-colonies of infected cells, embedded in a lawn of uninfected cells, were being visualised (see for example **Figure 1**).

Indeed, when infected cultures were diluted so that single colonies could be distinguished on the coverslips and were blotted and assayed for PrP^{Sc}, it was surprisingly found that the culture comprised PrP^{Sc} positive and negative colonies. The present invention is based upon this finding, together with the demonstration that the proportion of PrP^{Sc} positive colonies is a function of the prion titre of the sample used to infect the cells. We describe herein a quantitative prion assay which is based on these surprising findings.

With the cell line which we describe herein (N2a/Gary) it is possible to quantitatively determine prion concentrations as low as those that can be determined by endpoint titration in mice, and with greater precision. The assay can advantageously be completed in 2 weeks or less, as compared to 20 weeks in the most rapid mouse assay.

Thus, in one aspect, the invention provides a method for detecting a prion in a sample comprising
(i) providing a population of cells which are susceptible to prion infection
(ii) contacting the population of cells with the sample
(iii) incubating the cells on a growth substrate
(iv) assaying for prion colony formation
wherein presence of prion colonies indicates presence of prions in the sample.

Cells may advantageously be expanded after exposure to prions and prior to assaying for prion colony formation. Thus the invention provides a method as described above further comprising the step of incubating the cells and replating onto growth substrate. This step may advantageously remove the cells from the original solid phase sample. Thus the invention provides a method as described above wherein the step of incubating the cells on the growth substrate comprises separating the cells from the sample, and incubating the cells.

Furthermore, carry-over of sample can cause problems in the assay. This is particularly important when the sample comprises homogenate, because specks of particulate brain homogenate can contribute background readings when counting colonies. In order to overcome this problem, the invention advantageously provides for reduction of background sample carry-over by splitting and replating the cells after contact with the sample and before assaying for prion colony formation. Thus the invention provides a method as described above wherein incubating the cells on a growth substrate comprises the steps
(i) incubating the cells until they reach confluency
(ii) detaching the cells from the growth substrate
(iii) incubating the cells on fresh substrate at an initial density of approximately one tenth confluent density. The reduction of problematic background reduces with the introduction of this splitting step. Advantageously the background is further reduced by repeating the steps twice or three times or even more. After 2 splits 5-10% background can remain. After three splits, background is reduced to insignificant amounts. The optimal number of splitting steps is therefore three. Therefore the invention provides a method as described wherein steps (i)-(iii) are performed a further two times.

Assaying for prion colony formation may advantageously be achieved by monitoring PrPSc. Thus, in another embodiment, the invention provides a method as described above wherein assaying for colony formation comprises the steps
(i) transferring the cells to a support
(ii) visualising PrPSc
(iii) noting the presence or absence of colonies showing PrPSc.

Assaying for prion colony formation via PrPSc may be accomplished by first degrading PrPc, leaving only PrPSc which may be advantageously be easily detected using an anti-PrP antibody. Thus the invention provides a method as described above wherein assaying for colony formation comprises the steps
(i) transferring the cells to a nitrocellulose support
(ii) degrading PrPc by treating the cells with proteinase K
(iii) visualising PrPSc by staining with anti-PrP antibody
(iv) noting the presence or absence of colonies showing PrPSc.

It is convenient for the colony assay to produce the colonies on manipulable substrates. Advantageously these can be coverslips. Therefore the invention provides a method as described above wherein transferring the cells to a support comprises the steps
(i) detaching the cells from the growth substrate
(ii) seeding the cells onto coverslips
(iii) incubating the cells for about 4 days
(iv) blotting the cells onto the support

In another embodiment (such as the small colony or single cell embodiment (sometimes referred to as the 'scrapie cell' or 'SC' assay)), it is advantageous to assay the colonies using automated counting equipment. In this embodiment it is advantageous to apply the cells directly to the support without seeding onto coverslips and incubating. Thus the invention provides a method as described above wherein transferring the cells to a support comprises the steps detaching the cells from the growth susbstrate and applying the cells directly to the support. Advantageously the counting equipment may take the form of an ELISPOT reader. Accordingly the invention provides a method as described above wherein the support comprises an ELISPOT assay plate.

It is convenient to use coverslips to support the cells of the assay. Thus the invention provides a method as described above wherein the growth substrate is a coverslip comprising plastic or glass.

The colonies are easier to assay when they have reached a certain size. This is advantageously provided for by incubating the cells for 6-7 days on the growth substrate. Thus the invention provides a method as described above wherein incubation of the cells on the growth substrate is for approximately 6 to 7 days. The number of cells placed on the coverslip is important. If the proportion of infected colonies is expected to be small, it is possible to place up to about 130'000 cells on the coverslip and still be able to discern individual colonies, which will be small. If many colonies are expected, that number of cells would give a uniformly black layer, and one would place only 500 cells or less on the coverslip; the colonies are then larger. For unknown samples we therefore prepare several coverslips with varying cell numbers, from 500 to 130'000 cells per coverslip (13 mm diameter) for each sample. The number of colonies is usually about 40-50% of the number of cells plated ("plating efficiency"). The actual efficiency plays no role in the calculations as long as reference samples are run in parallel.

The sample is taken up efficiently by the cells in about 3 days. Thus the invention provides a method as described above wherein the cells are contacted with the sample for approximately 3 days.

It is an advantage of the present invention that prions may be assayed when present on solid phase substrates. Thus the invention provides a method as described above wherein the sample comprises solid phase material.

When assaying solid phase samples, it is advantageous to allow the cells to adhere to said solid phase samples to facilitate uptake of prion infectivity. Thus the invention provides a method as described above wherein the cells are contacted with the sample by allowing the cells to adhere to the solid phase material, and incubating for at least three days. Optionally the incubation time may be extended. Thus the invention provides a method as described above wherein the cells are incubated with the solid phase material for approximately 8 days.

As disclosed herein, the N2a/Gary cell line is advantageously especially sensitive to prion infection. Thus the invention provides a method as described above wherein cells are N2a/Gary cells. Furthermore, the invention provides an N2a/Gary cell line deposited as described hereinbelow.

N2a/Gary cells are particularly sensitive to infection by RML prions. Thus the invention provides a method as described above wherein the prion being detected is RML.

The invention further provides a method for quantification of prions in a sample comprising performing the method as described above wherein the step of assaying for prion colony formation further comprises the step of counting the number of colonies. Absolute determinations of prion number may advantageously be made by comparison with numbers obtained in the mouse assay, namely the LD₅₀ units. Thus the invention provides a method as described above further comprising estimating the number of prions in the sample by using the number of colonies counted to determine the number of prions present from a calibration curve. The calibration curve is advantageously a log-log curve as described hereinbelow. Thus the invention provides a method as described above wherein the calibration curve is a log-log plot.

Thus, the invention relates to methods for assaying prion infectivity. In particular, the invention relates to *in vitro* methods for assaying prion infectivity which advantageously alleviate the need to assay in animal models. Furthermore, the invention provides methods for quantitatively assaying prion infectivity *in vitro.* The invention further provides methods for calibrating quantitative assays for prion infectivity such as the log-log calibration methods described herein.

The invention advantageously provides methods for quantitatively assaying prions present on solid substrates.

The invention advantageously provides cell lines which facilitate the quantitative assay of prion infectivity. The invention also provides methods for producing cell lines for quantitative prion assay.

The methods of the present invention advantageously provide greater sensitivity to prions.

In contrast to prior art techniques, we do not evaluate "infection" by blackening of the film following a PrPSc blotting assay, but we advantageously quantify prion infectivity by counting colonies as described herein. This is advantageous because quantification is more precise and avoids problems associated with having to subtract a film background, which can limit sensitivity.

As will be apparent, the invention further provides a method of propagating prions comprising infecting a susceptible cell line with prions as described herein, and propagating said infected cell line.

### Detailed Description of the Invention

### Overview

We have isolated cell lines and developed procedures which allow infectivity assays about as sensitive as the endpoint titration assay in indicator mice, typically allowing detection of infectivity up to a 2 x 10⁻⁷ dilution of brain homogenate. It should be noted that although the criterion for determining whether or not cells are infected is the accumulation of protease-resistant PrP, what is in fact being measured is transmission of infectious agent to the cells.

We have isolated N2a sublines that are highly susceptible to prions such as RML scrapie prions as well as cell lines that are resistant. In the course of this work we observed that monolayers of chronically infected N2a cells, when blotted onto membranes and assayed for PrP^{Sc}, gave a speckled pattern, suggesting that micro-colonies of infected cells, embedded in a lawn of uninfected cells, were being displayed. Indeed, when infected cultures were diluted so that single colonies could be distinguished on the coverslips, it became clear that some were PrP^{Sc}-positive and others negative. We then determined that it was possible to identify individual PrP^{Sc} positive cells filtered off on a nitrocellulose membrane. We established conditions under which the proportion of PrP^{Sc}-positive cells was a linear function of the prion titre of the sample used to infect the cells and we developed a quantitative assay based on these findings. With the susceptible N2a/Gary cell line we can quantify prion concentrations about as low as those that can be determined by endpoint titration in mice. The assay can be completed in 2 weeks, as compared to 25 weeks in the most rapid mouse assay [Fischer, 1996 #3602], and at a fraction of the cost.

### Prion, PrPc and PrPSc

As used herein the term "prion" refers to a protein-containing infectious particle causing transmissible spongiform encephalopathy in an appropriate host.

PrPSc is a conformational isoform of PrPc (the normal form of prion protein) and is believed to be the main or only component of the prion.

### Visualisation of PrPSc

PrPSc may be visualised by any suitable means. For example; this may be by binding of a conformation-specific molecule to the PrP protein present to show up the PrPSc: A conformation-specific molecule may be an antibody molecule specific for PrPSc.

A preferred approach to visualisation of PrPSc is to remove PrPc for example by proteinase K digestion, and then use a PrP binding molecule such as an anti-PrP antibody to show up remaining PrPSc. (PrPSc is resistant to proteinase K digestion and so will be the only PrP molecule to be shown up by an anti-PrP antibody after proteinase K digestion.)

Preferred PrP antibodies include 6H4 and ICSM18. Secondary conjugates are chosen appropriately depending upon how the read-out (counting) is being performed.

### Cell Lines

The methods of the present invention may employ any suitable cell line. A suitable cell line is one which is susceptible to infection by the prions which are being assayed i.e. the cell line used must be capable of supporting a population of the prions which are being assayed. For example, when assaying RML prions the cell line used must be susceptible to RML prion infection. In principle, any cell line known to be susceptible to the prions being assayed may be employed in the methods of the present invention.

Cell lines suitable for use in the methods of the present invention may be selected for their prion susceptibility properties. For example, cell lines may be produced by the 'sib selection method'. A variant of this method of selecting cell lines for use in the present invention is presented in Example 5.

Example 5 sets out further possible prion/cell line combinations useful in the methods of the present invention.

In a preferred embodiment, the cell line used is N2a/Gary. In a highly preferred embodiment, the prions being assayed are RML prions and the cell line used is N2a/Gary.

### N2a/Gary Cell Line

The present invention provides the N2a/Gary cell line. This cell line has been derived from the parent cell line N2a which is available from the ATCC. The derivation is described in Example 1. N2a/Gary is surprisingly found to be far more sensitive to prion infection than prior art cell lines. N2a/Gary therefore possesses advantageous properties compared to prior art cell lines. N2a/Gary has been deposited with the ECACC in accordance with the Budapest Treaty.

### Biological Deposit

A deposit of N2a/Gary cells has been made with a depository institution in accordance with the Budapest Treaty.

The depository institution is the European Collection of Cell Cultures, having the address of Centre for Applied Microbiology and Research, Porton Down, Salisbury, Wiltshire, SP4 0JG, United Kingdom.

The depositor is the Medical Research Council (MRC) of 20 Park Crescent, London, W1B 1AL, United Kingdom.

The deposit was made on 13 March 2002.

The deposit was made before the filing date of this application.

The Accession number of this deposit is 02031337.

N2a cells are mouse neuroblastoma cells designated 'Neuro-2a' ('N2a') and are deposited with the ATCC having accession number CCL-131. Characteristics of N2a cells are known in the art. The N2a/Gary cell line is derived from the N2a parent as described herein.

The description of the method of making these N2a/Gary cells may be found herein, such as in Example 1.

The scientific name (i.e. taxonomic description) for N2a/Gary cells is *Mus musculus* subclone N2a/Gary. The description of the further characteristics of the N2a/Gary cells may be found throughout the description, in particular in the Examples section. The cells are useful in the methods of the present invention such as in the assay of prions and in the quantification of prions.

Cells can become infected with mycoplasma or other agents. Curing of mycoplasma is known to a person skilled in the art. Indeed, many service laboratories will perform this routine procedure for a nominal fee. Preferably cells used in the assays of the invention are mycoplasma free. A further deposit of N2a/Gary has been verified as mycoplasma free. The Accession number of this deposit 03031801. The remaining details are as above, except that the date of deposit if 18 March 2003.

### Population of Cells

A population of cells refers to a plurality of cells, in other words the term 'population' means more than one cell. In the methods of the present invention, the samples are contacted with a population of cells and then the number of cells which became infected with prions is estimated via the colony/cell blot assay, or "Colony Assay" for short. The term colony assay includes the small colony or single cell colony assay which is discussed in more detail below (sometimes called 'scrapie cell' or 'SC' assay). Exact numbers of cells which make up a population will depend on the exact formulation of the assay which should be expected to vary according to the exact format used for the assay, the surface area available on the growth substrates, the size of the sample to be contacted or other factors. Examples of optimal numbers of cells to use in different assay formats may be found in the Examples section. In 24-well plates, a population of approximately 100,000 cells per well in 1ml of medium may be used. When the sample is a liquid or suspension, it is convenient to use a cell monolayer as a population of cells. Clearly, the exact number of cells which are present in a monolayer population varies depending upon the area of monolayer used and upon the density of the cells of the monolayer. It is convenient to use cell monolayers in 96-well plates. When the sample comprises a disc such as a steel disc or a plastic (e.g. Thermanox (trade mark)) disc, a population of cells would comprise a number dependent on the surface area of the disc. This number should be chosen such that the cells are seeded onto the disc at a density allowing for about 8 days' growth. This number will plainly vary according to the growth rate of the cells, and is easily estimated by a person skilled in the handling of cultured cells. Appropriate numbers range from about 100 to 1000 cells per cm². Varying the particular sizes of cell populations to use in particular assay situations is well within the ability of the skilled man in the art with reference to the guidance presented herein.

In a preferred embodiment, the population of cells comprises a single cell type. Preferably, the population of cells is a population of N2a/Gary cells.

### Contact with the sample

Contacting of the sample with a population of cells may take any form which results in viable cells coming into association with the sample, allowing the adsorption of prions should any be present in the sample. Contacting of the cells with the sample may take the form of adding the sample to the cell medium, or may take the form of seeding the cells onto the sample when the sample is in the solid phase. The seeding of the cells is preferred when the sample is a wire or a disc. However, it will be apparent that the wire or disc may equally be introduced to a population of cells which could expand onto the surfaces of the disc or wire, thereby alleviating the need to seed the cells.

It will be apparent that the contacting of the sample with a population of cells may not necessarily involve the contact of the sample with each single cell in the population, but involves contact with the population generally and will therefore involve contact with a number of cell(s) comprised by said population. For example, when the sample is a disc or wire or other solid phase material, it is to be expected that only a proportion of the population will adhere to the solid phase sample, thereby contacting it. Other cells may contact the solid phase sample, and then go on to adhere to other surfaces. Clearly, when the sample comprises homogenate, it will be apparent to a person skilled in the art that in such an embodiment substantially the whole population of cells may be contacted. However, contacting of the sample with a population of cells will ordinarily mean contact with the population generally as explained above.

### Sample

The sample may be any entity capable of harbouring prions. The methods of the present invention may advantageously be applied to a wide range of forms of sample. For example, the sample may be in the form of a brain homogenate, a metal device such as a metal wire, a solid disc such as a plastic or glass disc or any other suitable form contactable with a population of cells.

### Support

In conducting the colony assay, it is advantageous to transfer the cells to a support for the visualisation of PrPSc. The support may be any support capable of retaining the molecules of interest (PrP molecules). Depending upon the format of the assay, different forms of support may be preferred in different circumstances such as to ensure ease of handling or to meet other physical requirements. In a preferred embodiment, the support comprises nitrocellulose such as a nitrocellulose membrane. Cells may be transferred to the support by any suitable method. A preferred method is cell blotting where nitrocellulose membrane is overlaid onto the cells, incubated, and then removed from the substrate, resulting in the transfer of cells onto the support. After the transfer, the membrane is dried, preferably for 1 hour at 40°C. An even more preferred method is direct application of cells onto ELISPOT plates such as those comprising Immobilon-P as support, which is particularly advantageous for automated counting applications.

### Transfer of cells to growth substrate

When assaying for prion colony formation, the cells are incubated with a growth substrate. In those embodiments of the invention in which the cells are contacted with the sample before being placed onto the growth substrate, then the cells are transferred to the growth substrate after the contact with the sample. The exact manner of this transfer will vary according to the individual circumstances or format of the assay. For example, the transfer step may simply comprise allowing the cells to expand from their existing substrate onto the growth substrate. This form of transfer is preferred when the sample is a metal wire. In this embodiment, cells are contacted with the metal wire sample and are allowed to adhere to the sample (the wire). Transfer is then effected by placing this wire onto a growth substrate and incubating, when the natural migration and expansion of the cells results in transfer from the wire to the growth substrate.

In another embodiment, the cells may be removed from the existing substrate and replated ('seeded') onto the growth substrate. This form of transfer is preferred when the existing substrate is a disc of plastic, metal or glass. The cells may be removed/detached from the existing substrate by any suitable means such as rinsing them off the substrate, trypsinisation or other such mode of detachment. The resulting cell suspension is then contacted with the growth substrate, completing the transfer. In another embodiment, cells may be grown on prion-coated plastic, metal or glass surfaces for several days and blotted directly onto a nitrocellulose membrane.

### Separation of cells from the sample

Separation of the cells from the sample after contact with the sample may be desirable in some embodiments, for example to facilitate conducting the assays of the present invention, such as when the sample comprises a wire. Separation may be by any suitable means known in the art.

Separation of cells from the sample may simply mean washing the cells, for example when the sample comprises brain homogenate the cells may be washed by repeated medium changes. Separation of cells from the sample may mean detachment such as when the sample comprises a disc or a wire. Furthermore, separation of the cells from the sample may mean simply allowing them to expand off the sample and thereby become separated such as when the sample comprises a wire which is then incubated with the growth substrate, thereby effecting separation.

It may be desirable to introduce a further incubation step between separating the cells from the sample and transferring them to the growth substrate. This may be advantageous for example to expand the numbers of cells. In this embodiment, the cells may be cultured, splitting and replating as necessary, for a period before transfer to the growth substrate for the colony assay. In a preferred embodiment, in particular when the sample is a metal wire, cells may optionally be incubated for a week before transfer to the growth substrate for the assay, or may be incubated for three weeks, or even more, before transfer to the growth substrate for the assay.

When undiluted brain homogenates containing much PrPSc are used to infect monolayers (for example in a search for susceptibility to BSE prions), enough PrPSc may cling to the monolayer to give a positive reaction for PrPSc without the cells actually having been infected. This "false positive" effect can be monitored by using in parallel a monolayer of non-susceptible cells. If such a control layer shows PrPSc "specks", it means that the monolayers of homogenate-exposed susceptible and non-susceptible cells must be split eg. 1:5, preferably 1:10, and again grown to monolayers and assayed. This process must be continued until the original PrPSc has been diluted out and there are no "specks" in the negative control. Then the persistence of PrPSc in the blot assay means that cells have been infected. Preferably three rounds of 1:10 splitting and regrowth are used.

### Growth substrate

The assays of the present invention involve incubating cells on a growth substrate to form colonies. The growth substrate may be any substrate suitable for the cells being used in the assay. For example, the growth substrate may comprise glass or plastic or derivatised plastics. In a preferred embodiment, the growth substrate comprises 25mm plastic coverslips placed in 6 well tissue culture plates.

### Calibration curve

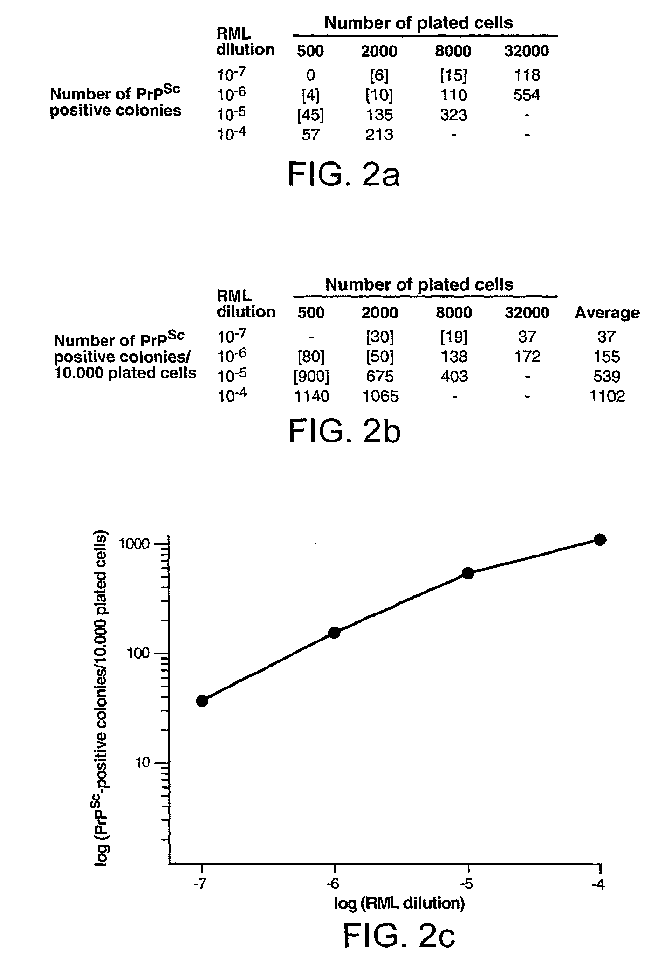

Calibration curves are prepared by assaying known amounts of prions in samples using the assays of the present invention. The quantification may be related to an animal infectivity assay by titration of the samples in known animal models such as the mouse test system. Absolute quantification may not be required in an embodiment of the invention in which samples are merely compared. In this case, relative quantification will be sufficient, such as by comparing numbers of colonies for given amounts of sample. In other embodiments, absolute quantification by preparation of and reference to a calibration curve may advantageously be conducted. For more details, see the Examples section including Figure 2c which presents a typical calibration curve for quantification.

Example 2, experiment 2, correlates the in vivo mouse prion assay with the Colony Assay.

### Colonies

The colonies mentioned in the methods of the present invention are small sub-populations or clones of cells which are infected with prions. It has been surprisingly found that, when prion susceptible cells are exposed to prions, that a subset of them may become infected. These infected cells multiply and retain prions, accumulating PrPSc in the process. These cells effectively form individual pools or clones of infected cells which remain in close association due to the adherent nature of the cell lines used and the format of the assays described herein. In this manner, small colonies of infected cells may be observed by visualising the PrPSc amongst the whole cell background or monolayer. This PrPSc is surprisingly found in small patches or spots which correspond to expansion of prion-infected sub-populations of cells (i.e. colonies, preferably discrete colonies). It is these spot-like patches or colonies which the assays of the present invention are designed to optimise and to quantify. These colonies have a granular form, having a dot-like or fleck-like appearance, similar to small discs or spots. When larger numbers of colonies are present, distinguishing of individual colonies can be more difficult as the edges of discrete colonies may merge or come to border one another. This may advantageously be avoided by plating them at a lower density on the growth substrate. Examples of the more detailed appearance and morphology of these colonies may be found in the accompanying Figures, see for example Figures 1, 3 and 5. Colonies may be quantified by counting. This may be optimised by incubating the cells on the growth substrate at densities which best suit quantification. This may be determined by trial and error, with reference to the Examples section which presents preferred densities.

In a preferred embodiment, prion colony formation is assayed by the 'small colony' or 'single cell' colony assay (SC-assay). In this embodiment, colonies are advantageously assayed at an early stage. Cells/colonies can even be assayed directly before outgrowth into a large cell colony. In this embodiment, a 'colony' may comprise a very small number of cells and may even comprise only a single cell.

Quantification, calibration and other techniques described herein are equally applicable to the 'small colony' (or 'single cell') embodiment of the invention.

The choice of manual counting versus automated counting, or whether to grow out the colonies or to assay them early or as single cells, can be made by the person skilled in the art depending upon his needs and/or the equipment available at the time of the assay. It will be appreciated that the SC assay has advantages of speed, high throughput and automation, whereas the colony assay with outgrowth and blotting of colonies is less complicated to perform and can be carried out with cheaper equipment, and may be preferred for analysis of small sample numbers.

The present invention embraces, but is not limited to, cell lines sensitive to human, bovine, ovine, murine, non-human primate, goat, mule deer, feline and hamster prions, such as described in Example 5.

The invention further relates to methods of transferring infectivity to susceptible cell clones using prions immobilised or attached to surfaces of metals, plastics, glass.

The invention further relates to methods for assaying tissues for prions by transiently inserting wires or needles consisting of metal, plastic and glass, and then assaying these wires or needles by the colony assay methods described herein.

The present invention describes methods for assaying prion infectivity adsorbed to or associated with solid substrates. The methods shown in the Examples sections involve direct contact of the cells of the assay with the solid substrate. Without wishing to be bound by theory, it is possible that the prions are not irreversibly bound to the solid substrates, but are capable of desorbing and diffusing. In this case, it will be apparent to a person skilled in the art that the assays may be performed avoiding direct contact of the cells with the solid substrate being tested. For example, the assays may be performed by juxtaposing the cells and the substrate a very small distance apart, such as a few microns apart, and then performing the assay. Furthermore, it may be possible to transfer the infectivity from one solid substrate to another by a similar juxtaposition of the two substrates. The substrate to which the infectivity had been transferred may then be assayed via direct contact or via juxtaposition as explained above.

### Sample carry-over

Because scrapie-infected brain homogenate contains PrP^{Sc} particles that can attach to the N2a cell monolayer and give background signals similar to signals from scrapie-infected cells, the cultures may advantageously be split 1:10 a number of times, preferably three times, to dilute out extraneous particles. The effectiveness of this procedure may be monitored by culturing in the presence and absence of an antibody to PrP that prevents accumulation of PrP^{Sc} [Enari, 2001 #5573] or by exposing both susceptible and "non-susceptible" cells to the prion sample. Under the conditions we describe, the scrapie-positive "spots" detected after exposure of susceptible cells to prions followed by three 1:10 splits, are virtually entirely due to *de novo* infected cells, demonstrating excellent reduction of background by the splitting steps.

It should be noted that continuous treatment with PrP antibody of cells overexpressing PrP 10-20 fold, even in the absence of infection, may cause accumulation of "spots" that persists after low-level proteinase K treatment, perhaps due to endocytosis and aggregation of PrP-antibody complexes. This phenomenon can be prevented by removing the antibody 24 h before the assay.

### Counting of Colonies

Colonies are advantageously quantified by counting. Counting may be by any means such as by eye or by automated means described herein.

In one embodiment of the colony assay, the cells, after forming a monolayer subsequent to the optional advantageous third split to reduce backgound, are plated on coverslips, allowed to develop to microcolonies and transferred to membranes. After PK treatment, PrP^{Sc}-positive microcolonies are visualized by a chemiluminescent immunoassay and the resulting spots recorded on X ray film are counted by eye. This procedure requires no sophisticated equipment.

However the procedure may be advantageously automated. The small colony (single cell or scrapie cell (SC)) assay is based on the finding that single PrP^{Sc}-positive cells, when filtered off on an Elispot membrane, can be identified by an enzyme-linked immunoassay for PrP. The assay can be performed in a 96-well plate format and up to about 2000 "spots" per well can be resolved under the microscope and counted automatically by appropriate Elispot scanning equipment in a few minutes. The response is considered linear between 0-2000. The most robust linear response is found between 0-1000.

The linear range of the assay, as applied to dilutions of brain homogenates from terminal, RML-infected mice, is between 2 x 10⁻⁷ and 10⁻⁵ while the dynamic response extends to 10⁻⁴. At lower dilutions there is no further increase in spot numbers, and, without wishing to be bound by theory, it would seem that some component(s) of the homogenate, present also in uninfected brain, may be inhibitory to the assay.

The SC assay, from start (preparation of the monolayer) to finish (colony counting), takes 14 days. The mouse infectivity assay, using *tg*a*20* mice (PrP-overexpressing mice with shortened incubation times), takes at least 140 days [Fischer, 1996 #3602].

The problems inherent to quantification of the infectivity assay in the mouse are set forth in [McLean, 2000 #5612]. We estimate that the costs of assaying a single prion sample by end-point titration, with use of 30 mice as in **Table 2**, are about £450 (*7.5 p*/*day*/*mouse; 200 days x 30 mice x 0.075 BP*), as compared with the expenditure of about £2 per sample for the SC assay (6 wells/sample; £30 per plate; without amortisation of equipment). It is likely that the SC assay can replace a large proportion of the in vivo assays for prions and thereby contribute to the well-being of mice worldwide.

### Further applications

GT1 and PC12 lines (Butler et al., 1988; Markovits et al., 1981; Race et al., 1987; Schätzl et al., 1997), epithelial rabbit cell line (Vilette et al., 2001) and the mesodermal cell line (SMB cell line) may each be used in the present invention. Susceptible cell lines may be selected *via* prion challenge as described herein. These susceptible cell lines may then be used in the assays of the present invention. These applications are particularly advantageous in the assay of infectivity of other prion species, for example prion species which may not readily infect N2a cell lines.

The present invention will now be described by way of Example, but is not to be regarded as limited to those embodiments described in the Examples. The Examples make reference to the following drawings.

### Brief Description Of The Drawings

**Figure 1****.** Colony assay on serially diluted RML-infected brain homogenate. Twenty thousand N2a Gary cells were plated into the wells of a 96-well plate, in Optimem-10% FCS. After 24h the medium was replaced by 200 microliter of RML brain homogenate at the dilution indicated (10⁻¹=10% brain homogenate). The 10% homogenate was prepared from terminally ill, RML-scrapie-infected mice. The dilutions were made into 0.01% uninfected brain homogenate to prevent adsorption losses. After 3 days the cell layers were washed twice with Optimem-10% FCS and transferred into the wells of a 24-well plate. After another 4 days the cells were suspended and the number of cells indicated were placed onto a 25-mm coverslip. After 7 days the colonies were transferred to a nitrocellulose membrane and PrP^{Sc}-positive colonies were determined. (a) 32'000 cells plated gave 33 and 1 colony at 10⁻⁷ and 10⁻⁸ dilution, respectively (b) 128'000 cells plated gave 121 and 17 colonies at 10⁻⁷ and 10⁻⁸ dilution, respectively.
**Figure 2****.** Dose-response of the colony assay. N2a/Gary cells were exposed to serial dilutions of RML-infected brain homogenates as described in the legend to Figure 1. 500, 2000, 8'000 and 32'000 cells were plated on 25mm coverslips for each dilution and after 7 days subjected to the blotting assay (a) The number of PrP^{Sc}-positive colonies as a function of the number of cells plated. Colony counts below 50 (in brackets) were not used for the further calculations (b) The data from (a) recalculated per 10'000 cells. (c) double-logarithmic plot of the number of colonies/10'000 cells versus the dilution of RML (from b) gives a straight line.

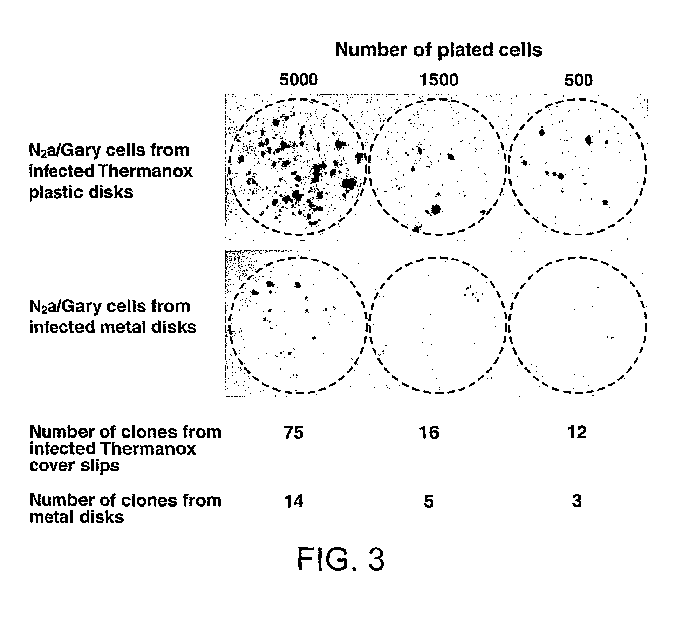

**Figure 3****.** Transfer of prions from metal and plastic discs to N2a/Gary cells. Metal discs (10mm diameter, washed for 1 hour with 100% Triton X-100 and extensively rinsed) and coverslips (Nunc thermanox, 13 mm diameter) were shaken overnight with 200 ul 1% RML in a 24-well plate, washed 5 times with 1 ml PBS and air-dried. The discs were placed in the wells of a 24-well plate, approx. 8000 N2a/Gary cells in 1 ml of Optimem-10% FCS were added and allowed to adhere. After 8 days the cells were rinsed off the surface of the discs, counted and 500, 1500 and 5000 cells were deposited on 25-mm Thermanox coverslips. After 1 week the colonies were transferred to nitrocellulose filters and PrPSc-positive colonies identified.

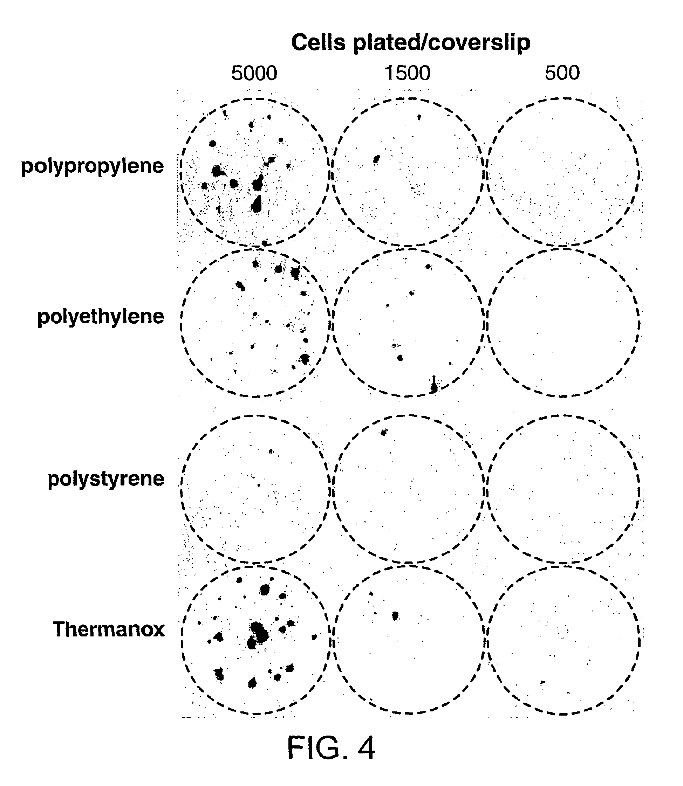

**Figure 4****.** Transfer of prions from different plastic surfaces to N2a/Gary cells. Plastic discs (polypropylene, polyethylene, polystyrene and Thermanox, 13 mm diameter) were shaken overnight with 200 microliters 1% RML in the wells of a 24-well plate, washed 5 times with 1 ml PBS and air-dried. The discs were placed in the wells of a 96-well plate and 8'500 N2a/gary cells in 1 ml of Optimem-10% FCS were added and allowed to adhere. After 4 days the cells were rinsed off the surface, counted, and 500, 1500 and 5000 cells were deposited on 25-mm Thermanox coverslips. After 1 week the colonies were transferred to nitrocellulose filters and PrPSc-positive colonies were identified as described herein.

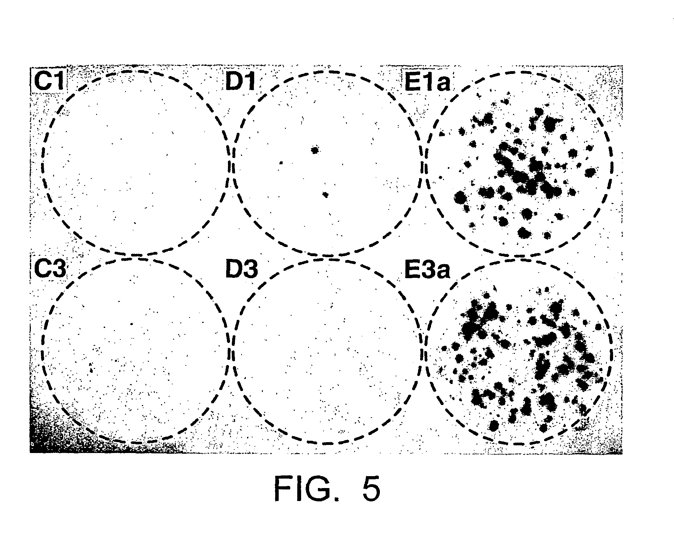

**Figure 5****.** Colony assay of prions adhering to steel wires "dipped" for 5 min into brains of mice at different times after infection. The figure shows the data for the 32'000 cell platings of wires dipped 41 days (C1 and C3), 61 days (D1 and D3) and 140 days ( E1a, E3a) after inoculation of CD1 mice.

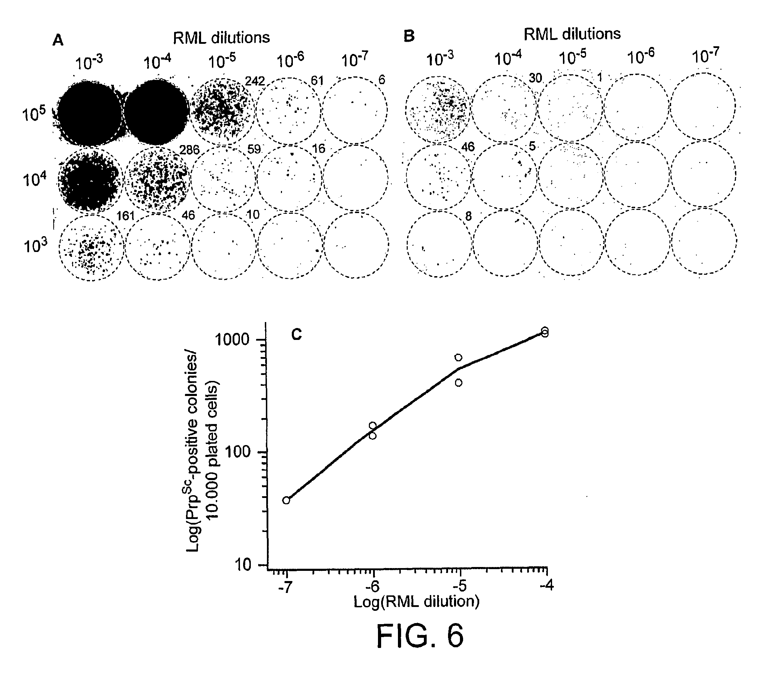

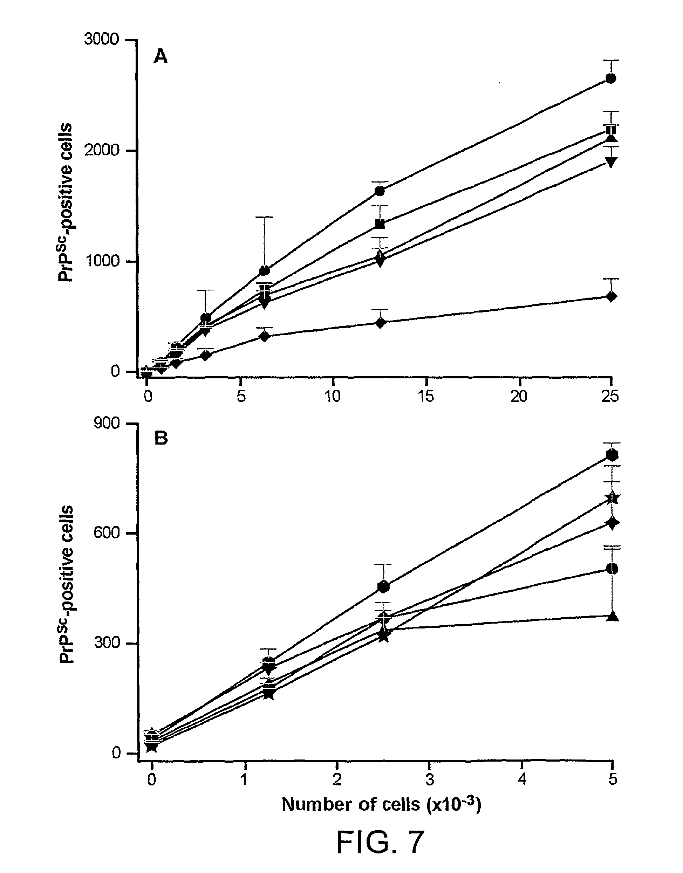

**Fig. 6****: Dose-response relationship between the dilution of infected RML homogenate and the number of PrP^{Sc}-positive colonies as determined by the Colony Assay.** 2 x 10⁴ susceptible N2a cells (N2a/Gary) (**A**) and "non-susceptible" cells (N2aR33) (**B**) were incubated with various dilutions of RML (I2424, see **Table 2**) in Optimem-10% FCS, as indicated. After 3 days the cells were suspended, split 1:10 and grown for 3 days. After 2 two more splits, 10⁵, 10⁴ and 10³ cells were mixed with non-infected N2a/Gary cells to give a final number of 10⁵ cells and plated onto 25-mm Thermanox coverslips. After 4 days of growth, the cells were transferred onto nitrocellulose membranes and the number of PrP^{Sc}-positive colonies was determined as described herein. The cell number on the membranes was kept constant because the extent of proteinase K digestion is dependent on the amount of protein on the membrane (see **Figure 7**) (**C**) Double-logarithmic plot of RML dilution versus number of PrP^{Sc}-positive colonies per 10.000 plated cells. The assay was carried out in duplicate.
**Fig. 7****: Optimisation of proteinase K concentration for the Scrapie Cell assay.** Varying numbers of chronically infected N₂a cells were filtered onto the membranes of an Elispot plate. The total number of cells was adjusted to 25'000 (a) or 5'000 (b) cells with uninfected N2a/Gary cells.
   After drying at 50°C for 1 h the samples were incubated with 50ul lysis buffer containing varying concentrations of proteinase K (Fig 7a: circle (0.5 microgramm PK/ml), square (1 microgramm PK/ml), triangle (0 microgramm PK/ml), inverted triangle (2 microgramm PK/ml), diamond (5 microgramm PK/ml); Fig 7b: hexagon (0.25 microgramm PK/ml), 5-point star (0.375 microgramm PK/ml), 4-point star (0.125 microgramm PK/ml), circle (0.5 microgramm PK/ml), triangle (1 microgramm PK/ml)) for 90 min at 37°C and processed as described in the Examples. Mean values of triplicate samples ± SD are shown.

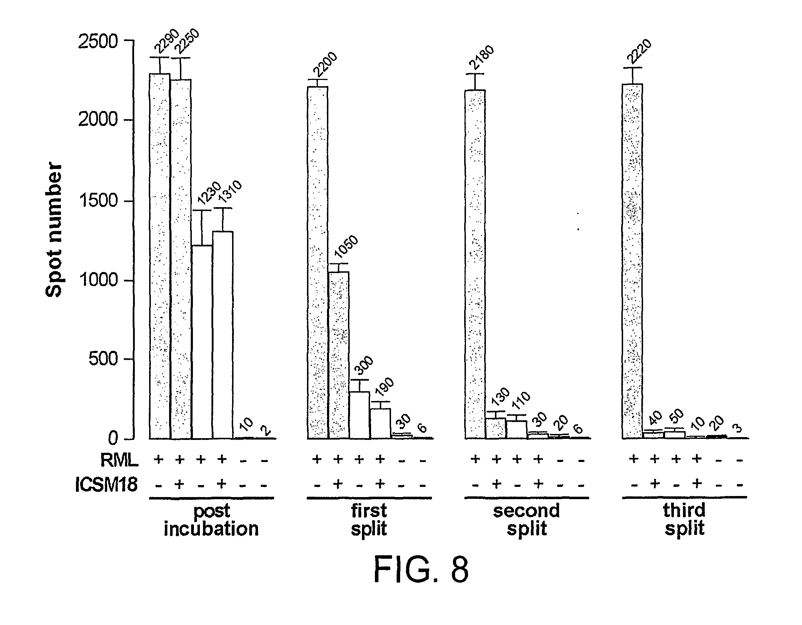

**Fig. 8****: Three successive splits are optimal to dilute out PrP^{Sc} particles from the initial inoculum.** 2 x 10⁴ susceptible N2a cells ((N2a/Gary (PD88) gray bars) and "non-susceptible" NN2a cells (white bars) were incubated with a 10⁻⁴ dilution of RML (I2424) in Optimem-10% FCS, in the presence or absence of 5ug/ml anti-PrP antibody ICSM 18 as indicated. As controls, cells were incubated with Optimem-10% FCS alone (in the absence of RML and ICSM 18). Three days after infection the cells were suspended, an aliquot of 25'000 cells was filtered off and the number of PrP^{Sc}-positive spots was determined by the small colony assay (Scrapie Cell or single cell assay); both susceptible and non-susceptible cells give rise to PrP^{Sc} positive signals and treatment with PrP antibody, which prevents de novo PrP^{Sc} accumulation, did not diminish the number of spots, showing that all spots can be accounted for by pre-existing PrP^{Sc} particles. After three 1:10 splits the non-susceptible cells as well as the susceptible cells treated with antibody showed less than 2% of the spots given by the susceptible cells. The number of spots for non-infected N2a/Gary cells and N2aR33 cells, the fifth and sixth bar of each group, respectively, represent the background. Mean values of complicates ± SD are shown.

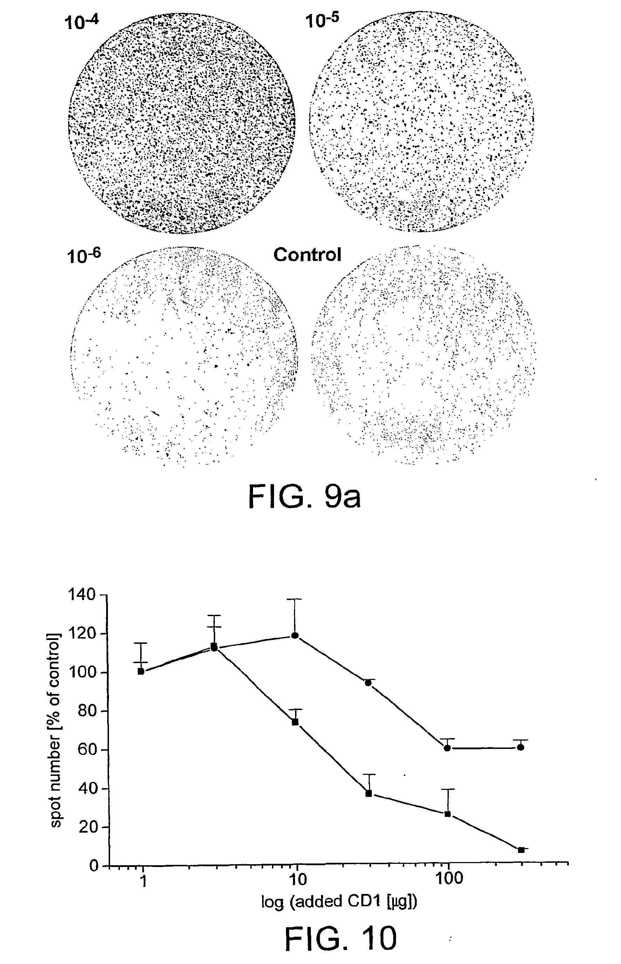

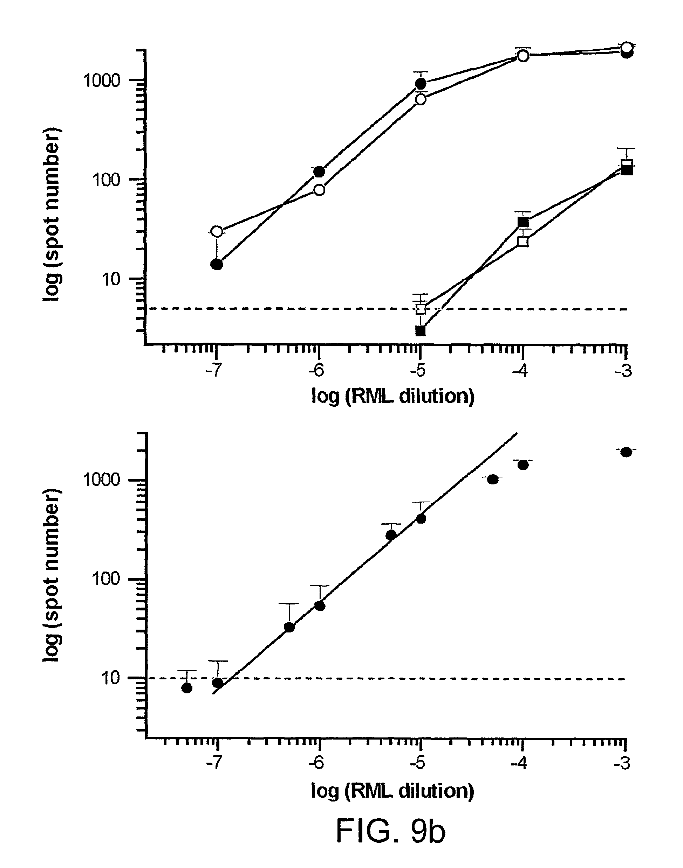

**Fig. 9****: Dose-response relationship between dilution of RML homogenate and the number of PrP^{Sc}-positive cells as determined by the Scrapie Cell assay. 2 x 10⁴** susceptible N2a/Gary cells (N2aPD88, N2aPD112) and "non-susceptible" cells (N2aR33 and N2aATCC) were incubated for 3 days with various dilutions of RML (12424) in Optimem-10% FCS as indicated. The cells were split three times 1:10, aliquots of 25'000 cells were filtered into wells of an Elispot plate and the number of PrP^{Sc}-positive cells was determined by the Small Colony assay. **A.** Representative wells of an Elispot plate showing spots given by N2a/Gary cells exposed to various dilutions of RML I2424 or to medium (control). **9B**. Double-logarithmic representation of the number of "spots" versus the RML dilution for susceptible N2a/Gary cells (closed circles PD88), susceptible N2a/Gary cells (open circles PD112), "non-susceptible" N2aR33 cells (closed squares) and "non-susceptible" N2aATCC cells (open circles, upper chart). "Non-susceptible" cells are about 3 logs less susceptible than the susceptible cells. The lower chart shows a separate assay using N2a/Gary cells (solid circle) with the linear response range, 5 x 10⁻⁶ to 10⁻⁵, indicated. Mean values of quadruplicate measurements ± SD are shown. The number of background spots given by 25.000 non-infected N2a/Gary cells is depicted by a dashed line.
**Fig. 10****: Inhibitory effect of non-infected homogenate.**
   2 x 10⁴ susceptible N2a/Gary cells were incubated for 3 days with 300 cl of RML (12424) dilutions (10⁻⁴, circles and 10⁻⁵, squares ie. solid circle = 10-4 dilution of RML); solid square = 10-5 dilution of RML)) in Optimem-10% FCS with increasing amounts of non-infected CD1 homogenate as indicated. The cells were split three times 1:10, aliquots of 25'000 cells were filtered into wells of an Elispot plate and the number of spots was determined by the small colony/single cell assay (SC assay). The maximal inhibition of spot number was determined with an addition of 300 µg CD1 homogenate (corresponding to a 10⁻² dilution). The number of spots are expressed as percentage of the values for the lowest amount of added CD1 (1 µg), corresponding to a 3 x 10⁻⁵ dilution. Mean values of quadruplicate measurements ± SD are shown. fig 10

### Examples

### Example 1: Isolation of a N2a-derived cell line highly sensitive to RML prions.

Sixty-five individual N2a clones from a ATTC-derived laboratory stock were screened for sensitivity to RML prions essentially as described (Bosque and Prusiner, 2000). Six susceptible, 11 weakly susceptible and 48 non-susceptible clones were identified. A clone designated as N2a/Gary was found to be two orders of magnitude more sensitive to RML prions than the previously isolated, RML-sensitive clone designated N2a/Bos2 (Enari et al., 2001). The N2a/Bos2 clone had been selected from an N2a cell culture transfected with a PrP expression plasmid, but it did not overexpress PrP (Enari et al., 2001).

We have isolated an N2a cell line, named N2a/Gary, which is orders of magnitude more sensitive to RML scrapie prions in brain homogenates than the lines previously described (Bosque and Prusiner, 2000; Enari et al., 2001). Surprisingly, when carried out as described herein, the prion Colony Assay using N2a Gary cells is at least as sensitive as the endpoint titration assay in indicator mice, and an order of magnitude faster as well as several orders of magnitude cheaper. It should be noted that although the criterion for determining whether or not susceptible cells are infected is the accumulation of protease-resistant PrP, what is in fact being measured in our colony assay is transmission of infectious agent from the sample to the cells.

This N2a/Gary cell line is useful in the cell culture assays for prion infectivity ('colony assays') described herein.

### Example 2: Assay of mouse scrapie-infected brain homogenate by the colony assay

### Example 2 - Experiment 1:

A 10% homogenate of scrapie-infected brain from a terminally scrapie-sick mouse was serially diluted into PBS to a concentration of 0.01%, and, beyond that dilution, into 0.01% uninfected mouse brain homogenate as carrier, to avoid loss of infectivity by adsorption to the walls of tubes and pipette tips.

A semi-confluent monolayer of prion-susceptible cells was prepared by seeding 20,000 N2a/Gary cells in wells of 96-well plates and incubating for 24 hours. These were contacted with the sample (homogenate) by exposure for 3 days to 0.2 ml of the various dilutions of homogenate. After washing, the cells were transferred into the wells of a 24-well plate and cultured for another 4 days (this step may be shortened without diminishing the colony yield by more than 50%). The cells were counted and 2'000, 8'000, 32'000 and 128'000 cells were incubated on a growth substrate by plating on plastic coverslips and after 7 days assayed for prion colony formation by first transferring to nitrocellulose membranes; then PrP^{Sc}-positive colonies were identified by treatment with proteinase K and staining with PrP antibody (**Figure 1**).

In slightly more detail, colonies were transferred to a nitrocellulose membrane, dried at 40 °C for 1 h, treated with 5 microgram proteinase K/ml, denatured, blocked for 1 h with Superblock blocking agent (Pierce, Rockford Ill, USA), immunostained with antibody 6H4 followed by horseradish peroxidase-conjugated goat anti-mouse IgG1, and visualized by enhanced chemiluminiscence (ECL kit, Pierce, Rockford III, USA).

The logarithm of the number of positive colonies per 10⁴ plated cells plotted against the dilution of the homogenate, on a log/log scale, gave a straight line (**Figure 2**). Clearly, this is useful as a calibration curve.

### Example 2 - Experiment 1a

This is conducted as Experiment 1, except that following contact with the sample after the washing step the cells are uncubated and split 1:10 three times before plating onto coverslips. This advantageously reduces background staining and improves the accuracy of the assay.

### Example 2 - Experiment 2:

### Correlation of Colony Units with LD₅₀ Units: Colony Assays performed on RML brain homogenates with known LD₅₀ titres.

RML brain homogenates 4.1 and 5.0 with known LD₅₀ titres were a gift of Professor Adriano Aguzzi, Institute of Neuropathology, Kantonsspital Zurich. Both had a titre of about 10⁹ LD₅₀ Units/ g brain.

Twenty thousand N2a/Gary cells in Optimem-10 % FCS were plated into the wells of a 96-well plate. After 24h the medium was replaced by 200 microliters of RML 4.1 and 5.0 brain homogenate (supplied as 1 % suspension in 0.32 M sucrose, centrifuged) at final dilutions of 10⁻⁴, 10⁻⁵ and 10⁻⁶ (10% homogenate = 10⁻¹). After 3 days the cells were washed twice with Optimem-10% FCS, resuspended and counted. Cells were then plated on 25-mm coverslips at cell numbers of 500, 2.000, 8.000 and 32.000. Optionally cells were split 1:10 and regrown three times before plating on coverslips to reduce background as described above. After 7 days the colonies were transferred to a nitrocellulose membrane and PrP^{Sc}-positive colonies were determined.

### PrPSc-pos. colonies/10.000 plated cells

| | **RML dilution** | | |
|---|---|---|---|
| | **10(-4)** | **10(-5)** | **10(-6)** |
| **RML 4.1** | 423 | 62 | 8 |
| **RML 5.0** | 380 | 33 | 7 |

The *sensitivities* of the Colony Assay and LD50 Mouse Assay are compared:

LD₅₀ Mouse Assay: In order to detect infectivity (semi-quantitatively) in brain homogenate, 10 mice must be injected with at least 1 LD₅₀ unit of infectivity each. 1 LD₅₀ unit has to be contained in 30 microliters of sample, that is 33 LD₅₀ units per ml. Therefore a 10^{-6.5} dilution of the homogenate will give a positive result.

Colony Assay: Using the value of 400 colony units (i.e. positive colonies per 10'000 plated cells) resulting from a 10⁻⁴ dilution of the brain homogenate under the condition of our assay, and considering that at least 100'000 cells can be plated, 1 colony would result at a 10^{-7.6} dilution.

Thus, the *sensitivity* of the Colony Assay is at least as great and likely greater than that of the mouse assay.

The *equivalence of Colony Units and LD₅₀ Units* is examined:

In the example given above, a mouse brain contains 10⁹LD₅₀ Units per g. As regards Colony Units, considering that 200 microliters of brain homogenate at a 10⁻⁴ dilution result in about 400 colonies/ 10'000 cells (400 Colony Units), there are 10^{7.3} Colony Units / g brain (5 x 400 x 10⁴). Thus, one Colony Unit is equivalent to about 50 LD₅₀ Units (10⁹ /10^{7.3}). Note that the Colony Unit is defined as number of colonies per 10'000 cells plated.

### Time factors:

As carried out in this example, the Colony Assay, from start (preparation of the monolayer) to finish (colony counting), took 11 days. The End Point Assay using tg20 mice (these are the mice with the shortest incubation time available to us) takes at least 120 days (Fischer et al., 1996). The problems inherent to the Incubation Time Assay in the mouse, when applied to low-titre samples, are set forth in McLean and Bostock (2000).

In summary, the Colony Assay is at least as sensitive as the LD₅₀ End-point Assay in the mouse, at least ten times faster and orders of magnitude cheaper.

### Example 3: Quantification by the colony assay of prions bound to solid substrates

We have shown earlier that metal devices such as steel or gold wires exposed to scrapie-infected brain homogenates or inserted for as little as 5 minutes into intact brain bind prions tightly, as shown by the fact that after exhaustive washing they efficiently cause scrapie when inserted into brains of indicator mice (Flechsig et al., 2001). Typically, a steel wire inserted transiently into a scrapie-infected brain causes disease in an indicator mouse as efficiently as 20µl 1% homogenate from the same brain (Flechsig et al., 2001).

We now show that not only metal but also plastic surfaces (and even others which may include glass) bind prions tightly and transmit infectivity to adherent susceptible cells such as N2a/Gary cells.

The sample is in the form of steel discs (FE240310 AISI 316 Stainless Steel discs, Goodfellow Cambridge Limited) and plastic discs (Nunc Thermanox coverslips, consisting of a modified polyester) which were exposed to various dilutions of scrapie-infected brain homogenate, washed exhaustively with PBS and contacted with a population of prion susceptible cells (N2a/Gary cells) by seeding with N2a/Gary cells. After 8 days the cells were detached from the surfaces and 500, 1'500 or 5'000 cells and were incubated on a growth substrate by being plated onto coverslips. After 7 days formation of PrP^{Sc}-positive microcolonies (prion colony formation) was determined by the blotting assay. **Figure 3** shows that after exposure to 1% brain homogenate, 14 PrP^{SC}-positive colonies/5000 cells were found for the metal disks and 75/5000 cells for the plastic coverslips. In further assays we demonstrate that polypropylene, Thermanox and to a lesser degree polystyrene efficiently bound prions and transferred infectivity to adhering N2a/Gary cells (**Figure 4**).

### Example 4: Intravital determination of prion infectivity using the colony assay on dipped wires

In order to determine prion levels in mouse brain it has been hitherto necessary to sacrifice the mouse and titer the brain homogenate. Transient wire insertion ("wire dipping") into the brain of an anaesthetised mouse followed by assessment of infectivity by permanent implantation into an indicator mouse now makes it possible to determine prion levels in the same mouse at different times, for example to monitor the effect of experimental treatments. Because of the long incubation times, even in transgenic indicator mice strains overexpressing PrP, it is useful to measure prions attached to dipped wires or needles by the much more rapid colony assay provided by the present invention.

CD1 mice were intracerebrally inoculated with RML scrapie prions and culled 19, 25, 41 and 61 days after infection. As positive control, terminally sick CD1 mice were used that had been culled 140 days after intracerebral inoculation with RML prions. The sample comprised steel wire segments (5 x 0.15 mm) which were inserted for 5 min into brain and spleen of scrapie-infected CD1 mice culled at the various time points. After exhaustive washing of the wires with PBS, the amount of surface-bound infectivity was analysed by permanent insertion into indicator mice and by the colony assay. The brain and spleen that had been used for dipping were homogenised and assayed by the colony assay. Table 1 and Figure 5 show that the colony assay of the dipped wires was strongly positive by 140 days after infection, with 40-50 colonies per 10⁴ cells plated; the Colony Assay of the homogenate gave 2.4 x 10⁷ Colony Units/g brain. The infectivity titer of brain homogenate at 140 days after infection, as measured in indicator mice (Büeler et al., 1993) is around 10^{8.6} LD₅₀ units/10% brain homogenate. At 40 and 60 days the colony assay of the dipped wires was at the detection limit, with about 10 colonies per 10⁵ cells plated. The colony assay of the 40-day homogenate was negative; the 60-day homogenate gave 2 x 10⁶ Colony Units/g brain.

The infectivity titre of a homogenate determined in indicator mice at 56 days after infection was about 10^{5.4} LD₅₀ units/ml 10% brain homogenate, which means that the detection of 10 LD₅₀ units requires about 4 µg brain tissue. Because 30ul of a 1% homogenate, i.e. about 300 µg of brain tissue, can be readily injected intracerebrally, the limit of detection is in theory about 100 times higher than with the wire assay. Because plastic surface is about 5 times more efficient than steel surface in transferring prions to susceptible cells, the use of plastic needles rather than steel wire should increase the efficiency of the assay considerably.

**Table 1. Colony assay of wires "dipped" into brains of mice at various times after infection**

| | **Time points after infection (days)** | | | | | |
|---|---|---|---|---|---|---|
| **Cells analysed** | | | | | | |
| | 41 | | 61 | | 140 | |
| | **C1** | **C3** | **D1** | **D3** | **E1a** | **E3a** |
| 2.000 | 0 | 0 | 0 | 0 | 6 | 2 |
| 8.000 | 0 | 0 | 0 | 0 | 32 | 45 |
| 32.000 | 0 | 4 | 3 | 0 | 122 | 145 |
| 128.000 | 0 | 28 | 19 | 0 | >>> | >>> |

At the times after inoculation indicated, wire segments were inserted into brains of culled mice, washed, placed into wells of a 24 well plate and incubated with 1 ml of a cell suspension containing 100.000 N2a/Gary cells per ml of OPTIMEM medium. After 3 days, the wires were transferred into new wells (ca. 300 cells had attached to each wire) containing 1 ml of fresh OPTIMEM medium and cultured for another 6 days. After growth to confluency, cells were split twice a week at a dilution of 1:7.5, for a total of 3 weeks. To determine the number of PrP^{Sc}-positive cells N2a/Gary cells were plated at different cell amounts and grown to colonies. Cell suspensions of 2.000 - 8.000 - 32.000 and 128.000 cells were plated onto plastic coverslips (25 mm diameter) in 6 well plates. After 6 days of growth the number of PrPSc-positive colonies was determined by the cell blot assay.

### Cell Blot Assay

Colonies were transferred to a nitrocellulose membrane, dried at 40 °C for 1 h, treated with proteinase K, denatured, blocked for 1 h with Superblock blocking agent (Pierce, Rockford Ill, USA), immunostained with antibody 6H4 followed by horseradish peroxidase-conjugated goat anti-mouse IgG1, and visualized by enhanced chemiluminiscence (ECL kit, Pierce, Rockford III, USA).

### Example 5: Application to BSE and CJD

It has been reported that clones of N2a and Gut1-7 cells, transfected with a mouse PrP-expression plasmid and overproducing PrP, are susceptible to 22L, 139A and Chandler strains but not to 22A or 87V strains (Nishida et al., 2000). These authors suggest that overproduction of PrP is important for susceptibility to prions. Bosque and Prusiner selected untransformed N2a and GT1-trk sublines and found lines that were susceptible to RML but not to 139A (in contrast to the findings of Nisihida et al.), 301V or ME7 prions. Enari et al. (Enari et al., 2001) transfected N2a cells with a PrP expression plasmid and identified RML-sensitive sublines; interestingly, the most sensitive sublines did not overexpress PrP, while some resistant lines did. This suggests that prion susceptibility is a trait independent of PrP expression level, and that a population of uncloned N2a cells such as those used in prior art techniques contains cells some of which express the trait and others not. Without wishing to be bound by theory, PrP expression in a susceptible cell may further increase its susceptibility to prions or the level of PrP^{Sc} expression.

To apply the colony assay to BSE, CJD and other prion strains, cell lines susceptible to these prion strains are identified as follows. It is to be expected that the susceptible cell should contain a PrP with the sequence of the prion donor but as suggested above, a further "susceptibility trait" may be required. Cell lines susceptible to prions can be sought by the "sib selection method". In this approach, several pools of a defined number of cells are exposed to the prion strain of interest, grown for several doublings with splitting and assayed by the blotting assay (Bosque and Prusiner, 2000). When a positive pool is identified, subpools containing smaller numbers of cells are assayed by the same procedure. This results in a progressive enrichment of susceptible cells, finally yielding a clonal, susceptible population.

For example, if an initial cell population contains 1:1000 susceptible cells, the following strategy can be adopted:
Cycle 1. Twenty pools of 200 cells (to give on average 4 positive pools) are each grown to about 20'000-30'000 cells and 3 aliquots of about 5000 cells are frozen away. Two aliquots of 2-3000 cells are expanded to about 20'000 cells and exposed to prions for 3 days, cultured for 2 weeks and 20'000 cells are plated in duplicate on large coverslips for PrP^{Sc} blotting, to identify pools containing infected cells and to determine the number of positive colonies.
Cycle 2. A positive pool should now contain not less than 1:200 susceptible cells. The frozen sample of the pool that had given the largest number of positive colonies is thawed and 20 pools of 40 cells are grown to about 20'000-30'000 cells each, and processed as above. There should be on average 4 positive pools
Cycle 3. A positive pool should have 1:40 positive cells. The frozen sample of the pool giving the largest number of positive colonies is thawed and 20 pools of 10 cells are processed as above. This should give about 5 positive pools.
Cycle 4. A positive pool should have 1 in 10 positive cells. From the most positive pool, plate out 50 single cells, grow to 20'000-30'000 cells and assay aliquots of each clone as above.

It is of course possible to start with a larger number of pools or with pools containing a larger number of cells.

Cell lines used in this method can be neuroectodermal-derived from a variety of species, epithelial, immortalised embryonic brain cells, or other lines may yield the lines of desired susceptibility.

Combined with the dipping assay it is thus possible to detect prions by a minimally invasive procedure applicable to cattle, sheep, humans and other animals. PrPSc (indicative of prion infectivity) has been detected not only in brain, but also in tonsils of humans and sheep suffering from prion disease.

In this Example, the colony assay is performed as set out in the previous examples, substituting these cell lines for the N2a/Gary cell line as appropriate.

### Example 6: Preparation of cell lines with different susceptibilities to prion infection

### Overview

The neuroblastoma cell line N2a is moderately susceptible to infection by some strains of mouse scrapie prions, as evidenced by accumulation of infectivity and PrP^{Sc}. We have isolated highly susceptible N2a sublines and developed an in vitro quantitative assay for prion infectivity based on a colony or cell counting procedure. Susceptible N2a cells are exposed to prions for 3 days, split three times 1:10 and the proportion of PrP^{Sc}-containing cells is determined by an immuno assay. The dynamic range extends over 3 logs of prion concentrations and is linear over 2 logs. The assay is about as sensitive as the mouse inoculation assay, an order of magnitude faster and several orders of magnitude less expensive.

### Subcloning, propagation and storage of N2a cells.

N2a cells are routinely cultured in Optimem-FCS. After subcloning and identifying highly susceptible subclones [Enari, 2001 #5573], cells are expanded for about three passages. N2a/Gary (N2aPD) cells were isolated as described, by subcloning N2a cells [Enari, 2001 #5573]. By further subcloning and challenging with a 10⁻⁶ dilution of RML, clones N2aPD112 and N2aPD88 were generated giving the highest response by the SC assay were chosen as susceptible lines (*also designated N2alGary*).

Clones not showing PrP^{Sc} accumulation after exposure to a 10⁻⁴ RML dilution, such as N2aR33, were chosen as "non-susceptible" sublines. The chosen clones were expanded for about three passages and 50 aliquots each were frozen at -80 °C in freezing medium (Optimem-FCS, 6% DMSO) using a freezing container ("Mr. Frosty" (Nalgene) and stored in liquid nitrogen. Working stocks are not propagated for more than about 4 passages in order to avoid possible deterioration of susceptibility. Chronically infected cells were obtained after 6 subsequent 1:10 splits of freshly infected N2a/Gary cells with a 10⁻⁴ dilution of RML (12424).

### Isolation of N2a-derived cell lines highly susceptible or resistant to RML prions.

Sixty-five N2a clones from an ATTC stock culture were screened individually for susceptibility to RML prions. Monolayers in 96-well plates were exposed to RML brain homogenate, washed and passaged 3 times, splitting 1: 10 each time. The final monolayer was assayed for PrP^{Sc} by the blotting procedure, essentially as described [Bosque, 2000 #4993]. Six susceptible, 11 weakly susceptible and 48 apparently non-susceptible clones were identified. A clone designated as N2aPD was about 2 orders of magnitude more sensitive to RML prions than the non-cloned ATTC stock. A second round of subcloning yielded cell line N2a/Gary that is 3 orders of magnitude more sensitive than non-cloned cells (see below, **Figure 9b**). Several "non-susceptible" lines were isolated by exposure to higher concentrations of RML homogenates and selection for cells showing little or no PrPSc accumulation ; clone N2aR33 was about 3 orders of magnitude less sensitive than N2a/Gary cells (**Figure 6a**).

### Example 7: Colony assay for prion infectivity

### Background

We noticed that when confluent cell layers that had been exposed to dilute prion preparations were subjected to the cell blot assay, a spotty pattern resulted, suggesting the presence of individual PrP^{Sc}-positive micro-colonies, embedded in a background of uninfected cells. This indeed proved to be the case and this surprising finding permitted the development of the quantitative *in vitro* prion infectivity assays of the present invention.

### Colony Assay using N2a/Gary cells

ATTC N2a cells, susceptible N2a/Gary cells and "non-susceptible" N2aR33 cells were exposed in parallel to various dilutions (10⁻³ to 10⁻⁸) of infected brain homogenate, split successively three times 1:10 to dilute out PrP^{Sc} carried over from the inoculum (see below). After reaching confluence, 10⁵, 10⁴ or 10³ cells were mixed with non-infected N2aPD cells to give a total of 10⁵ cells, plated on a coverslip, grown for 4 days and subjected to the cell blot immunoassay [Bosque, 2000 #4993]. As shown in **Figure 6a**, the number of positive colonies was about proportional to the number of cells plated and decreased linearly with increasing dilution of the inoculum. While N2a/Gary cells exposed to 10⁻⁵ RML-infected brain homogenate gave 240 positive colonies per 100'000 plated cells, "non-susceptible" cells gave 6 positive colonies; these were either due to a low infection rate or to residual carry-over of PrP^{Sc} from the inoculum. As shown below, "non-susceptible" cells were indeed very weakly susceptible to infection.

**Figure 6b** shows that the number of colonies is an almost linear function of the RML brain homogenate dilution from 10⁻⁷ to 10⁻⁴ (10% brain homogenate = 10⁻¹). In this experiment the infectivity of the brain homogenate was 10⁹LD₅₀ units / g brain, and ten LD₅₀ units gave rise to about 1.2 scrapie-positive colony per 100'000 cells plated. Using a reference curve such as that of **Figure 6B** it is possible to determine the infectivity of an unknown sample. This is an example of absolute quantification of infectivity according to the present invention.

### Example 8:

### The 'Small Colony' Or 'Single Cell' ("Scrapie-Cell") Assay For Prion Infectivity

### Outline

As demonstrated above, the colony assay is extremely useful for a analysing the infectivity of samples. However, it can be relatively labour-intensive (involving counting colonies visually) and can require plating the cells at different densities in order to optimise the number of colonies that can be resolved.

In this Example, it is demonstrated how to modify the assay to allow simultaneous processing of large numbers of samples and automated quantification.

### Detection of single, PrP^{Sc} positive cells by the "Elispot" technique

We ascertained that under appropriate circumstances, single PrP^{Sc}-positive cells can be visualised by an enzyme-linked immuno assay (ELISA). Therefore, rather than allowing infected cells to form colonies, we advantageously filtered defined numbers of cells onto the membranes of Elispot plates, subjected them to lysis and proteinase K (PK) digestion. PrP^{Sc}-positive cells were visualised using a monoclonal PrP antibody and alkaline-phosphatase-linked anti-IgG1 antiserum. Positive cells were counted using the Zeiss KS Elispot system and Wellscan software.

A critical parameter for optimal sensitivity and signal-to-noise ratio is the PK concentration relative to the number of cells deposited on the membrane. We prepared mixtures of chronically scrapie-infected and uninfected N2a cells in different proportions, filtered off a total of 25'000 cells (about the maximum possible without reducing the flow-rate through the membrane) and 5'000 cells, respectively, and determined the PK concentration optimal for obtaining a high signal to noise ratio and a linear relationship between the number of cells applied to the filter and the number of positive "spots" counted (**Figure 7**).

Uninfected cells, even in the absence of PK treatment, gave no positive signal, perhaps because PrP^{C} was solubilised by the lysis buffer and washed through the filter. Interestingly, mild PK treatment improved the signal intensity and thereby increased the number of detectable infected cells somewhat. Excessive levels of PK decreased the number of spots, because, as shown previously, the resistance of PrP^{Sc} is not absolute [Kuczius, 1999 #4646][Büeler, 1994 #6252]. Under the conditions of our assay, the optimal PK concentrations for 5000 and 25'000 cells, respectively, were 0.25 and 0.5 µg/ ml for 90 min. Optimal values for intermediate numbers of cells may be determined by the person skilled in the art with reference to these figures.

The dynamic range of the assay extended up to about 2000 positive cells (over a background of typically 20-40 "spots"), when 25'000 cells were applied to the filter.

### The Infectivity Assay Scored By The Elispot Technique

After exposing susceptible cells to scrapie-infected brain homogenates it is advantageous to split the infected cultures three times 1:10 to dilute out PrP^{Sc}-positive particles present in the homogenates, as shown in the experiment of Figure 8. Twenty thousand susceptible (N2a/Gary (PD88)) and "non-susceptible" (NN2a) cells, respectively, were plated in the wells of a 96-well plate, cultured for 12 h and exposed to a 10⁻⁴ dilution of RML-infected brain homogenate for 3 days. The cells were then suspended and split 1:10. After 3 days confluence was reached and the cells were again split 1:10. This procedure was repeated once more. At each step, after reaching confluence, 25'000 cells were filtered onto the membranes of an Elispot plate, in octuplicate. In parallel, infection as well as all following steps, were carried out in the presence of a monoclonal PrP antibody (ICSM 18, [White, 2003 #6289], Beringue et al.) under conditions that prevent accumulation of PrP^{Sc} in chronically scrapie-infected N2a cells in the presence of Mab 6H4 [Enari, 2001 #5573]. Three days after infection the number of immunoreactive "spots", about 2'200, was similar whether or not PrP^{Sc} accumulation had been inhibited by PrP antibodies, both in susceptible and non-susceptible cells (**Figure 8**), showing that at this stage the vast majority of spots was due to pre-existent PrP^{Sc}. After the third split the spot number in the antibody-treated samples diminished to background levels, as compared to a value of about 2'200 spots in its absence, showing that the original PrP^{Sc} particles had been diluted out and/or degraded completely. Similarly, "non-susceptible" cells exposed to RML and split 3 times 1:10 typically led to a reduction of "spots" to about 50, less than 2.5% of the original level. This value was further reduced to 10 when PrP antibody was present in the medium, suggesting that "non-susceptible" cells were susceptible to infection, albeit at very low efficiency. Thus, the scrapie-positive "spots" detected after exposure of susceptible cells to prions followed by three 1:10 splits, are virtually entirely due to *de novo* infected cells.

### Dose-Response Calibration Of The Assay

We determined the dose response to prions, in this example RML prions. Susceptible and "non-susceptible" N2a cells, respectively, were exposed to RML-infected brain homogenates diluted 10⁻⁷ to 10⁻³ in Optimem-10% FCS and processed as above. **Figure 9a** depicts Elispot filters of samples at various dilutions and **Figure 9b** a plot of the automatically evaluated data, showing linearity between 2 x 10⁻⁷ and 10⁻⁵, and dynamic response up to 10⁻⁴.

### Optimising Further Parameters of The Colony Assay For Prion Infectivity

*Cell density of monolayer*: Monolayers of susceptible N2a cells were prepared by plating 10⁴, 2x10⁴ and 4x10⁴ per well and growing them. Monolayers were exposed for 3 days to diluted RML-infected brain homogenate as described and analysed by the SC assay. In this manner optimal cell densities for infection (ie. contact with sample) are determined.

### Time of exposure to infectivity:

Monolayers prepared by plating 2x10⁴ susceptible N2a cells were exposed to dilution of RML-infected brain for 1, 2 and 3 days and the SC assay was carried out as above. In this manner optimal times for exposure for infection (ie. contact with sample) are determined.

### Effect of uninfected brain homogenate on "SC spot" yield:

The finding that the spot yield at 10-4 and 10-3 dilutions were similar suggested inhibition of infection efficiency by a component of the homogenate. Increasing amounts of uninfected CD 1 brain homogenate were added to 10⁻⁴, 10⁻⁵ or 10⁻⁶ dilutions (in Optimem-FCS) of homogenates of scrapie-infected brains (RML2121, 2.95 x 10⁸ LD₅₀ units/g) and spot yields were determined by the SC assay. As shown in **Figure 10**, there is a progressive loss of "spots" as uninfected brain homogenate is added. With 10⁻⁵ RML, the spot number is halved at a concentration of 330 µg homogenised brain tissue/ml. The invention thus provides the use of homogenised uninfected brain tissue as a suppressor of prion infection. Furthermore, invention provides the use of homogenised uninfected brain tissue in the manufacture of a medicament for the suppression of prion infection.

### Example 9:

### Comparison of the mouse endpoint titration assay and the scrapie cell assay:

The brain homogenate of a terminally ill, RML-infected CD1 mouse (12424) was serially tenfold diluted and 30-µl aliquots of each dilution were injected i.c. into each of five *Tg*a*20* mice. In parallel, 100-µl aliquots were administered i.p. As shown in **Table 2**, all of 5 mice succumbed to scrapie when injected i.c. with dilutions down to 10⁻⁶, but only 3 of 5 mice at a 10⁻⁷ dilution (10% brain homogenate = 10⁻¹ dilution) and none of 5 mice after inoculation with a 10⁻⁸ dilution. The same brain homogenate, when assayed by the SC assay yielded 26 ± 23 "spots" per 25'000 cells ("SC units") for the 10⁻⁷ dilution (background = 1 ± 1 spots). The results are thus significantly above background for the 10⁻⁷ dilution (p= 0.03). By these criteria, it is demonstrated that the mouse infectivity assay and the SC assay have about the same sensitivity.

**Table 2. Infectivity assay of RML-scrapie-infected brain homogenate**

| Mouse Endpoint Assay⁺ | | | | **Scrapie Cell Assay^{#}** | |
|---|---|---|---|---|---|
| | | | | | |
| **Sample dilution *** | **Inocul ation** | **Scrapie sick /total** | **Incubation time (days ± S.D.)** | **SC Units ± S.D. (background)** | |
| | | | | | |
| 10⁻² | i.c. | 5/5 | 61 ± 3 | n.d. | |
| 10⁻⁴ | i.c. | 5/5 | 74 ± 5 | 2171 ± 268 | (15 ± 16) |
| 10⁻⁵ | i.c. | 5/5 | 88 ± 9 | 1039 ± 224 | (3 ± 2) |
| 10⁻⁶ | i.c. | 4/5 | 102 ± 12 | 75 ± 35 | (1 ± 1) |
| 10⁻⁷ | i.c. | 3/5 | 116 ± 27 | 26 ± 23 | (1 ± 1) |
| 10⁻⁸ | i.c. | 0/5 | >155 | 7 ± 6 | (3 ± 1) |
| 10⁻² | i.p. | 5/5 | 92 ± 3 | - | |
| 10⁻⁴ | i.p. | 5/5 | 116 ± 4 | - | |
| 10⁻⁵ | i.p. | 3/5 | 123 ± 16 | - | |
| 10⁻⁶ | i.p. | 0/5 | >155 | - | |

| | | | | | |
|---|---|---|---|---|---|
| *Ten brains of RML-infected, terminally sick CD1 mice were homogenised in PBS, using a sterile glass homogeniser. The 10 % (w/v) homogenate (designated 12424) was stored at -80°C. For the inoculation experiments, serial 10-fold dilutions of the 10 % (w/v) brain homogenate (designated as 10⁻¹ dilution) were prepared with PBS or with PBS containing 0.01 % brain homogenate of uninfected CD1 mice, so that all samples (except the 10-2 dilution) had a final concentration of 0.01% brain homogenate. Thirty µl of the indicated dilutions were inoculated intracerebrally or 100 µl i.p. into *Tg*a*20* mice. For the SC assay, the 10% brain homogenate was diluted into Optimem-10% FCS. ⁺Inoculated mice were observed for clinical symptoms every second day by and culled after definitive onset of disease. The experiment was terminated at 155 days post inoculation. The dilution leading to 50% scrapie diseased mice following i.c. inoculation was 10^{-6.9} and for the i.p. inoculation 10^{-5.2}, as calculated following Reed and Munch [Reed, 1938 #3529]. The brain therefore contained 10^{6.9}/(0.03) = 10^{8.4} LD₅₀ units/g as assayed intracerebrally, and (10^{5.2})/(0.03) = 10^{6.7} LD₅₀ units/g as assayed after i.p. inoculation. ^{#}The values, positive cells per 25'000 plated cells, are averages of 12 independent measurements of each sample on sensitive (N2a/Gary (PD88)) cells (backgrounds not subtracted) and 6 measurements of the backgrounds on "non-susceptible" N2aATTC cells exposed to the same samples. The value for the 10⁻⁷ dilution, 26 ± 23 ( n= 12 ), was measured over a background of 1 ± 1 (n = 6); The probability that the value of the 10⁻⁷ sample does not differ from background is 0.03 and for the 10⁻⁸ sample, 0.01 (Student's unpaired t-test). | | | | | |

Considering that 300 µl of brain homogenate at a 10⁻⁵ dilution resulted in 1039 scrapie-positive-cells/ 25'000 cells (1039 SC units) and at a 10⁻⁶ dilution in 75 SC units, there are about 10^{8.47} SC units / g brain (3.3 x 1/2([1039 x 10⁵]+ [75 x 10⁶])). The mouse brain from which the same samples were derived contained 10^{8.38} LD₅₀ units/g. Thus, under the conditions of the experiment, one SC unit is equivalent to about 1.23 LD₅₀ unit (10^{8.47}/(10^{8.38}))*.* However, without wishing to be bound by theory, it should be mentioned that the SC units have been defined herein and moreover may depend on antibody avidity and concentrations as well as the parameters chosen for counting by the Elispot equipment. Therefore, in comparing results from independent assays, it is important to include a reference prion preparation, preferably a mouse-titered scrapie-brain homogenate.

**Table 3** shows the results of SC assays performed on 3 further RML brain homogenates with known LD₅₀ titers.

**Table 3 Comparison of mouse and SC infectivity assays of various brain homogenates**

| | RML 4.0^{a} | RML 4.1^{a,b} | RML 5.0^{b} | RML I2424^{c} |
|---|---|---|---|---|
| **Mouse infectivity assay** (LD₅₀ units/g) | 10^{8.0} | 10^{8.86}(a), 10^{9.2}(b) | 10^{9.6} | 10^{8.4} |
| | | | | |

| **SC assay** (positive cells/25'000 cells) | | | | |
|---|---|---|---|---|
| Dilution 10⁻⁴ | 2152 ± 116 | 1863 ± 365 | 2353 ± 110 | 2171 ± 268 |
| Dilution 10⁻⁵ | 892 ± 85 | 916 ± 299 | 1541 ± 297 | 1039 ± 224 |
| Dilution 10⁻⁶ | 126 ± 33 | 132 ± 37 | 320 ± 147 | 75 ± 35 |
| | | | | |
| **SC units/g^{d}** | 3.5 x 10⁸ | 3.7 x 10⁸ | 7.8 x 10⁸ | 2.95 x 10⁸ |
| **SC units:LD₅₀ units** | 3.5 | 0.5(a), 0.2(b) | 0.2 | 1.2 |

| | | | | |
|---|---|---|---|---|
| All mouse infectivity determinations were done by the endpoint assay [Reed, 1938 #3529]; the in vitro assays were by the SC assay as described herein. ^{a} Ms. Petra Schwarz and A.Aguzzi ^{b} From [Thackray, 2002 #5704] ^{c} From Table 2 ^{d} Average of the 10⁻⁵ and 10⁻⁶ dilutions, multiplied by 3.3 | | | | |

The ratio of SC units to LD₅₀ units ranged from 0.2 to 3.5 for four independent brain homogenates prepared and assayed by the mouse assay in different laboratories; this spread is likely due to the imprecision of the mouse infectivity assay [McLean, 2000 #5612].

### Infectivities in homogenates of mouse brains at different times after inoculation determined by the mouse infectivity and the SC assay.

To further compare the sensitivity of the mouse infectivity and the SC assay, brain homogenates from wild type CD1 mice were assayed by both methods at different times after inoculation. As shown in **Table 4**, neither method detected infectivity at days 19, 25 and 41 after i.c. inoculation, but after 61 and 140 days, infectivity was detected by both methods.

**Table 4. Infectivity of brain homogenates at different times after inoculation, assayed by the mouse infectivity and SC assay**

| Mouse infectivity assay | | | | SC assay |
|---|---|---|---|---|
| Sample (days p.i., sample #) | dilution | sick/total | incubation time (days) | SC units ± s.d. |
| 19, *A1* | 10⁻² | 4/5 | 97 ± 9 | 26 ± 19 |
| | 10⁻³ | 3/5 | 96 ± 7 | 7 ± 6 |
| | | | | |
| 19, *A3* | 10⁻² | n.d. | n.d. | 10 ± 8 |
| | 10⁻³ | n.d. | n.d. | 18 ± 24 |
| | | | | |
| 25, *B1* | 10⁻² | 0/4 | >140 | 11 ± 4 |
| | 10⁻³ | 0/4 | >140 | 4 ± 3 |
| | 10⁻⁴ | 0/5 | >140 | n.d. |
| | | | | |
| 25, *B3* | 10⁻² | n.d. | n.d. | 6 ± 3 |
| | 10⁻³ | n.d. | n.d. | 4 ± 1 |
| | | | | |
| 41, *C1* | 10⁻² | 1/5 | 107 | 11 ± 7 |
| | 10⁻³ | 0/4 | >140 | 7 ± 5 |
| | 10⁻⁴ | 0/5 | >140 | 5 ± 2 |
| | | | | |
| 41, *C3* | 10⁻² | 0/5 | >140 | 6 ± 2 |
| | 10⁻³ | 0/3 | >140 | 18 ± 17 |
| | 10⁻⁴ | 0/4 | >140 | 8 ± 4 |
| | | | | |
| 61, *D1* | 10⁻² | n.d. | n.d. | 486 ± 53 |
| | 10⁻³ | 5/5 | 73 ± 2 | 381 ± 42 |
| | 10⁻⁴ | 5/5 | 77 ± 6 | 103 ± 21 |
| | 10⁻⁵ | 4/5 | 107 ± 6 | n.d. |
| | 10⁻⁶ | 4/5 | 91 ± 8 | n.d. |
| | | | | |
| 61, *D3* | 10⁻² | n.d | n.d | 471 ± 71 |
| | 10⁻³ | n.d | n.d | 553 ± 70 |
| | 10⁻⁴ | n.d | n.d | 235 ± 21 |
| | | | | |
| 140, *E1* | 10⁻³ | 3/3 | 56 ± 5 | 859 ± 136 |
| | 10⁻⁴ | n.d | n.d | 858 ± 208 |
| | 10⁻⁵ | 5/5 | 84 ± 2 | n.d. |
| | 10⁻⁶ | 4/5 | 85 ± 3 | n.d. |
| | 10⁻⁷ | 4/5 | 103 ± 19 | n.d. |
| | 10⁻⁸ | 1/5 | 107 | n.d. |
| | | | | |
| 140, *E3* | 10⁻³ | n.d. | n.d. | 998 ± 190 |
| | 10⁻⁴ | n.d | n.d | 778 ± 91 |

CD1 mice were inoculated i.c. with 0.03ml RML I2424 (1%, about 7.5x10⁴ LD₅₀ units). After 19, 25, 41 and 61 days mice were culled; mice inoculated similarly were culled 140 d after infection as positive controls. Brain homogenates were prepared and mouse infectivity assays as described in **Table 2**. The background values for the SC assay were 9 ± 5 and were not subtracted. Dilutions are relative to brain tissue (10% = 10⁻¹). Positive values are in bold print.

### Example 10: The Colony Assay

Unless stated otherwise, all steps are carried out at room temperature. TBST (10mM Tris.HCl (pH 8.0), 150 mM NaCl, 0,1 % Tween 20) is prepared prior to use from a 10x stock solution. Twenty thousand susceptible N2a cells (doubling time about 24 h) in 200 µl Optimem (Invitrogen)-10% FCS (Invitrogen) (OFCS) are plated in wells of a 96-well plate. After 24 h at 37°C the medium is replaced by 0.3 ml sample in OFCS. After 3 days the confluent monolayer (about 10⁵ cells) is suspended by gentle pipetting. A 30-ul aliquot of each sample is placed into a well of 96-well plate containing 270µl OFCS and grown to confluency. The cells are split 1:10 two more times 1:10, as before.

After reaching confluency following the third split, the cells are suspended in 300µl OFCS and counted. Triplicate aliquots of 200, 1000, 5'000 and 25'000 cells are deposited onto 13-mm (or 24-mm) Thermanox coverslips (Nunc, Fisher Scientific UK) in 24-well (or 6-well) plates. After 4 days at 37°C, the colonies (plating efficiency about 40%) are blotted onto a nitrocellulose membrane (Trans-Blot Transfer Medium, Nitrocellulose, 0,45 µm, Bio-Rad Laboratories Ltd., Hemel Hempstead, Hertfordshire, UK ) soaked in lysis buffer (50 mM Tris-HCl (pH 7.4), 150 mM NaCl, 0.5 % sodium deoxycholate, 0.5 % Triton X-100). The membrane is dried for 1 h at 37°C, incubated in lysis buffer containing 5µg/ml proteinase K (specific activity 30 mAU/mg, Merck Pharmaceuticals, West Drayton, Middlesex, UK) for 90 min at 37 °C, rinsed twice with water and incubated in 2 mM PMSF (Sigma-Aldrich, Gillingham, Dorset, UK) for 10 min. The membrane is placed into 3 M guanidinium thiocyanate (>99%, Sigma-Aldrich), 10 mM Tris-HCl (pH 8.0) for 10 min, rinsed 5 times with water, incubated in Superblock (Pierce) (TBS) blocking buffer for 1 h, shaken with anti-PrP antibody 6H4 (Prionics AG, Zürich, Switzerland; 1:5.000) or ICSM18 (J.Collinge, Prion Unit; 1:5000) in TBST (10 mM Tris.HCl (pH 8.0), 150 mM NaCl, 0,1 % Tween 20), 1 % nonfat dry milk (Marvel)) for 1h, then washed with TBST 4 times for 5 min. The membrane is incubated with horseradish peroxidase-conjugated anti-mouse IgG1 antibody (Zymed Laboratories, South San Francisco, CA, USA; 1:7.500 in TBST, 1% nonfat dry milk) for 1 h. After washing 5 times for 5 min with 1 x TBST, membranes are dried on filter paper (proteins upside), soaked for 3 min with ECL reagent (Super Signal West Pico, Pierce Biotechnology, Rockford, IL, USA) and exposed to X ray film (Hyperfilm ECL, Amersham Biosciences Ltd., Chalfont Buckinghamshire, England). PrP^{Sc}-positive colonies are counted and expressed relative to the number of plated cells.

### Example 11

### The Scrapie Cell (SC) assay (also called the 'small colony' or 'single cell' assay)

All solutions are filtered and plates are protected from dust during the assay. TBST is prepared freshly before use from a 10x stock solution. Unless stated otherwise, all steps are carried out at room temperature. The same procedure as in Example 10 above is followed to infect and propagate cells.

After reaching confluency following the third split, the cells are then suspended in 250µl PBS, counted (about 500 cells/µl) and 25'000 or 5'000 cells are pipetted into each of at least 3 wells (containing 200 µl PBS) of an Elispot plate (Multi Screen Immobilon-P 96 well Filtration Plates, sterile, 0.45 µm; Millipore (U.K.) Ltd., Watford, England), activated with 70% ethanol and rinsed twice with PBS). Suction is applied to the Elispot filtering device and the plates are dried at 50°C for 1 h. To each well 50 µl proteinase K (optimised at 0.5 µg/ml in lysis buffer for 25'000 cells; 0.25 µg/ml for 5000 cells) are added and suctioned off after 90 min at 37°C. After washing with 160 µl PBS, the samples are exposed to 160 µl PMSF for 10 min and washed once with 160 µl PBS. Following incubation with 160 µl 3 M guanidinium thiocyanate-10 mM Tris-HCl (pH 8.0) for 10 min, the filters are washed 4 times with 160 µl 1x PBS by suction and incubated with 160µl Superblock for 45 min. Fifty µl PrP antibody ICSM18 (1:5000 in 1x TBST / 1% nonfat milk powder) are added and after 1 h the supernatant is suctioned off and the wells are washed 7 times with 160µl 1x TBST. Fifty µl anti-IgG1-AP (Southern Biotech, Birmingham, Alabama USA; 1:4'500 in 1x TBST / 1% milk powder) are added for 1 h. After washing 8 times with 160 µl 1x TBST the plates are dried. To each well 50µl Alkaline Phosphatase Conjugate Substrate (prepared as recommended by the manufacturer, Bio-Rad) are added for 8 min and the wells are washed twice with 160µl water. Plates are stored in the dark at 20°C until evaluation.

PrP^{Sc}-positive cells were counted using the Zeiss KS Elispot system (Stemi 2000-C stereo microscope equipped with a Hitachi HV-C20A colour camera, a KL 1500 LCD scanner and Wellscan software from Imaging Associates, Bicester, Oxfordshire, UK). The settings are optimised to give a maximal ratio of counts for wells with PrP^{Sc}-positive to those with negative control samples while keeping the sample counts as high as possible. Parameters are adjusted using the training feature of the program according to the instructions of the manufacturer.

### References

Birkett et al. (2001). Scrapie strains maintain biological phenotypes on propagation in a cell line in culture, EMBO J. 20, 3351-8.
Bosque, P. J., and Prusiner, S. B. (2000). Cultured cell sublines highly susceptible to prion infection, J. Virol. 74, 4377-4386.
Büeler, H., Aguzzi, A., Sailer, A., Greiner, R. A., Autenried, P., Aguet, M., and Weissmann, C. (1993). Mice devoid of PrP are resistant to scrapie, Cell 73, 1339-1347.
Butler, D. A., Scott, M. R., Bockman, J. M., Borchelt, D. R., Taraboulos, A., Hsiao, K. K., Kingsbury, D. T., and Prusiner, S. B. (1988). Scrapie-infected murine neuroblastoma cells produce protease- resistant prion proteins, J. Virol. 62, 1558-1564.
Enari, M., Flechsig, E., and Weissmann, C. (2001). Scrapie prion protein accumulation by scrapie-infected neuroblastoma cells abrogated by exposure to a prion protein antibody, Proc. Natl. Acad. Sci. USA 98, 9295-9.
Fischer, M., Rülicke, T., Raeber, A., Sailer, A., Moser, M., Oesch, B., Brandner, S., Aguzzi, A., and Weissmann, C. (1996). Prion protein (PrP) with amino-proximal deletions restoring susceptibility of PrP knockout mice to scrapie, EMBO J. 15, 1255-1264.
Flechsig, E., Hegyi, I., Enari, M., Schwarz, P., Collinge, J., and Weissmann, C. (2001). Transmission of scrapie by steel-surface-bound prions, Mol. Med. 7, 679-684.
McLean, A. R., and Bostock, C. J. (2000). Scrapie infections initiated at varying doses: an analysis of 117 titration experiments, Philos. Trans. R. Soc. Lond. B Biol. Sci. 355, 1043-50.
Manuelidis, L., Sklaviadis, T., and Manuelidis, E. E. (1987). Evidence suggesting that PrP is not the infectious agent in Creutzfeldt-Jakob disease, EMBO J. 6, 341-347.
Markovits, P., Dormont, D., Delpech, B., Court, L., and Latarjet, R. (1981). [Trials of in vitro propagation of the scrapie agent in mouse nerve cells], C. R. Seances Acad. Sci. III 293, 413-417.
McKinley, M. P., Meyer, R. K., Kenaga, L., Rahbar, F., Cotter, R., Serban, A., and Prusiner, S. B. (1991). Scrapie prion rod formation in vitro requires both detergent extraction and limited proteolysis, J. Virol. 65, 1340-1351.
Nishida, N., Harris, D. A., Vilette, D., Laude, H., Frobert, Y., Grassi, J., Casanova, D., Milhavet, O., and Lehmann, S. (2000). Successful transmission of three mouse-adapted scrapie strains to murine neuroblastoma cell lines overexpressing wild-type mouse prion protein, J. Virol. 74, 320-325.
Oesch, B., Westaway, D., Wälchli, M., McKinley, M. P., Kent, S. B., Aebersold, R., Barry, R. A., Tempst, P., Teplow, D. B., Hood, L. E., et al. (1985). A cellular gene encodes scrapie PrP 27-30 protein, Cell 40, 735-746.
Prusiner, S. B., Cochran, S. P., Groth, D. F., Downey, D. E., Bowman, K. A., and Martinez, H. M. (1982). Measurement of the scrapie agent using an incubation time interval assay, Ann. Neurol. 11, 353-8.
Race, R. E., Fadness, L. H., and Chesebro, B. (1987). Characterization of scrapie infection in mouse neuroblastoma cells, J. Gen. Virol. 68, 1391-1399.
Reed, J., and Muench, H. (1938). A simple method of estimating fifty per cent endpoints, Am. J. Hygiene 27, 493-497.
Sakaguchi, S., Katamine, S., Yamanouchi, K., Kishikawa, M., Moriuchi, R., Yasukawa, N., Doi, T., and Miyamoto, T. (1993). Kinetics of infectivity are dissociated from PrP accumulation in salivary glands of Creutzfeldt-Jakob disease agent-inoculated mice, J. Gen. Virol. 74, 2117-2123.
Schätzl, H. M., Laszlo, L., Holtzman, D. M., Tatzelt, J., DeArmond, S. J., Weiner, R. I., Mobley, W. C., and Prusiner, S. B. (1997). A hypothalamic neuronal cell line persistently infected with scrapie prions exhibits apoptosis, J. Virol. 71, 8821-8831.
Stahl, N., Baldwin,.M. A., Teplow, D. B., Hood, L., Gibson, B. W., Burlingame, A. L., and Prusiner, S. B. (1993). Structural studies of the scrapie prion protein using mass spectrometry and amino acid sequencing, Biochemistry 32, 1991-2002.
Vilette, D., Andreoletti, O., Archer, F., Madelaine, M. F., Vilotte, J. L., Lehmann, S., and Laude, H. (2001). Ex vivo propagation of infectious sheep scrapie agent in heterologous epithelial cells expressing ovine prion protein, Proc. Natl. Acad. Sci. USA 98, 4055-4059.

## Claims

1. A method for detecting a prion in a sample comprising
(i) providing a population of N2a/Gary cells which are susceptible to prion infection
(ii) contacting the population of cells with the sample
(iii) incubating the cells on a growth substrate
(iv) assaying for prion colony formation
wherein presence of prion colonies indicates presence of prions in the sample.

2. A method according to claim 1 wherein incubating the cells on a growth substrate comprises the steps
(i) incubating the cells until they reach confluency
(ii) detaching the cells from the growth substrate
(iii) incubating the cells on fresh substrate at an initial density of approximately one tenth confluent density

3. A method according to claim 2 wherein steps (i)-(iii) of claim 2 are performed a further two times.

4. A method according to any of claims 1 to 3 wherein assaying for colony formation comprises the steps
(i) transferring the cells to a support
(ii) visualising PrPSc
(iii) noting the presence or absence of colonies showing PrPSc.

5. A method according to any of claims 1 to 4 wherein assaying for colony formation comprises the steps
(i) transferring the cells to a nitrocellulose support
(ii) degrading PrPc by treating the cells with proteinase K
(iii) visualising PrPSc by staining with anti-PrP antibody
(iv) noting the presence or absence of colonies showing PrPSc.

6. A method according to any of claims 2 to 5 wherein transferring the cells to a support comprises the steps
(i) detaching the cells from the growth substrate
(ii) seeding the cells onto coverslips
(iii) incubating the cells for about 4 days
(iv) blotting the cells onto the support

7. A method according to any of claims 2 to 5 wherein transferring the cells to a support comprises the steps
(i) detaching the cells from the growth susbstrate
(ii) applying the cells directly to the support

8. A method according to claim 7 wherein the support comprises an ELISPOT assay plate.

9. A method according to any preceding claim wherein the growth substrate is a coverslip comprising plastic or glass.

10. A method according to any preceding claim wherein incubation of the cells on the growth substrate is for approximately 6 to 7 days.

11. A method according to any preceding claim wherein the cells are contacted with the sample for approximately 3 days.

12. A method according to any preceding claim wherein the sample comprises solid phase material.

13. A method according to claim 12 wherein the cells are contacted with the sample by allowing the cells to adhere to the solid phase material, and incubating for at least three days.

14. A method according to claim 13 wherein the cells are incubated with the solid phase material for approximately 8 days.

15. A method according to any one of claims 12 to 14 wherein the solid phase material comprises the growth substate.

16. A method according to any preceding claim wherein the prion being detected is RML.

17. A method for quantification of prions in a sample comprising performing the method according to any preceding claim wherein the step of assaying for prion colony formation further comprises the step of counting the number of colonies.

18. A method according to claim 17 further comprising estimating the number of prions in the sample by using the number of colonies counted to determine the number of prions present from a calibration curve.

19. A method according to claim 18 wherein the calibration curve is a log-log plot.

20. An N2a/Gary cell line deposited at the ECACC under accession number 02031337 or 03031801.

## Patentansprüche

1. Verfahren für den Nachweis eines Prions in einer Probe, wobei das Verfahren besteht aus:
(i) Bereitstellung einer Population von -N2a/Gary-Zellen, die empfänglich für Prioneninfektion sind,
(ii) Kontaktherstellung der Zellenpopulation mit der Probe
(iii) Bebrütung der Zellen auf einem Wachstumssubstrat,
(iv) Prüfung auf Bildung einer Prionenkolonie,
**dadurch gekennzeichnet, dass** das Vorkommen von Prionenkolonien auf das Vorkommen von Prionen in der Probe hinweist.

2. Verfahren gemäß Anspruch 1, **dadurch gekennzeichnet, dass** die Bebrütung der Zellen auf einem Wachstumssubstrat folgende Verfahrensschritte aufweist:
(i) Bebrütung der Zellen, bis sie Konfluenz erreichen,
(ii) Entnahme der Zellen aus dem Wachstumssubstrat,
(iii) Bebrütung der Zellen auf einem frischen Substrat bei einer Anfangsdichte von ca. einem Zehntel der Konfluenzdichte.

3. Verfahren gemäß Anspruch 2, **dadurch gekennzeichnet, dass**
die Verfahrensschritte (i) bis (iii) von Anspruch 2 noch zwei weitere Male durchgeführt werden.

4. Verfahren gemäß einem der Ansprüche 1 bis 3, **dadurch gekennzeichnet, dass** die Prüfung auf Koloniebilding folgende Verfahrensschritte aufweist:
(i) Übertragung der Zellen auf eine Unterlage,
(ii) visuelle Erkennung von PrPSc,
(iii) Feststellung des Vorkommens oder Nichtvorkommens von Kolonien, die PrPSc erkennen lassen.

5. Verfahren gemäß einem der Ansprüche 1 bis 4, **dadurch gekennzeichnet, dass** die Prüfung auf Koloniebildung folgende Verfahrensschritte aufweist:
(i) Übertragung der Zellen auf eine Nitrozellulose-Unterlage,
(ii) Degradierung von PrPc durch Behandlung der Zellen mit Proteinase K,
(iii) visuelle Erkennung von PrPSc durch Färbung mit Anti-PrP Antikörpern,
(iv) Feststellung des Vorkommens oder Nichtvorkommens von Kolonien mit PrPSc.

6. Verfahren gemäß einem der Ansprüche 2 bis 5, **dadurch gekennzeichnet, dass** die Übertragung der Zellen auf eine Unterlage folgende Verfahrensschritte aufweist:
(i) Abtrennung der Zellen vom Wachstumssubstrat,
(ii) Impfung der Zellen auf Deckgläser,
(iii) ca. 4 Tage lange Bebrütung der Zellen,
(iv) Blotting der Zellen auf die Unterlage.

7. Verfahren gemäß einem der Ansprüche 2 bis 5, **dadurch gekennzeichnet, dass** die Übertragung der Zellen auf eine Unterlage folgende Verfahrensschritte aufweist:
(i) Abtrennung der Zellen vom Wachstumssubstrat,
(ii) direktes Auftragen der Zellen auf die Unterlage.

8. Verfahren gemäß Anspruch 7, **dadurch gekennzeichnet, dass** die Unterlage eine ELISPOT-Assayplatte darstellt.

9. Verfahren gemäß einem der vorhergehenden Ansprüche, **dadurch gekennzeichnet, dass** das Wachstumssubstrat ein aus Plastik oder Glas bestehendes Deckglas darstellt.

10. Verfahren gemäß einem der vorhergehenden Ansprüche, **dadurch gekennzeichnet, dass** die Zellen ca. 6 bis 7 Tage lang auf dem Wachstumssubstrat bebrütet werden

11. Verfahren gemäß einem der vorhergehenden Ansprüche, **dadurch gekennzeichnet, dass** die Zellen ca. 3 Tage lang mit der Probe in Berührung bleiben.

12. Verfahren gemäß einem der vorhergehenden Ansprüche, **dadurch gekennzeichnet, dass** die Probe aus einem Festphasenmaterial besteht.

13. Verfahren gemäß Anspruch 12, **dadurch gekennzeichnet, dass** die Zellen mit der Probe **dadurch** in Berührung kommen, dass die Zellen am Festphasenmaterial anhaften und mindestens drei Tage lang bebrütet werden.

14. Verfahren gemäß Anspruch 13, **dadurch gekennzeichnet, dass** die Zellen mit dem Festphasenmaterial ca. acht Tage lang bebrütet werden.

15. Verfahren gemäß einem der Ansprüche 12 bis 14, **dadurch gekennzeichnet, dass** das Festphasenmaterial das Wachstumssubstrat darstellt.

16. Verfahren gemäß einem der vorhergehenden Ansprüche, **dadurch gekennzeichnet, dass** das nachgewiesene Prion RML darstellt.

17. Verfahren zur Quantifizierung von Prionen in einer Probe, **dadurch gekennzeichnet, dass** das Verfahren daraus besteht, dass es gemäß einem der vorhergehenden Ansprüche angewandt wird, wobei der Verfahrensschritt der Prüfung auf die Bildung einer Prionenkolonie weiterhin den Schritt enthält, die Anzahl der Kolonien zu zählen.

18. Verfahren gemäß Anspruch 17, **dadurch gekennzeichnet, dass** es weiterhin daraus besteht, die Anzahl der Prionen in der Probe **dadurch** zu schätzen, dass die Anzahl der gezählten Kolonien verwendet wird, um die Anzahl der Prionen mit Hilfe einer Kalibrierkurve festzustellen.

19. Verfahren gemäß Anspruch 18, **dadurch gekennzeichnet, dass** die Kalibrierkurve einen Log-Log-Plot darstellt.

20. N2a/Gary-Zellenlinie, die beim ECACC unter Zugangsnummer 02031337 oder 03031801 hinterlegt worden ist.

## Revendications

1. Procédé de détection d'un prion dans un échantillon comprenant les étapes consistant à :
(i) fournir une population de cellules N2a/Gary, qui sont susceptibles d'être infectées par un prion,
(ii) mettre en contact la population de cellules avec l'échantillon,
(iii) mettre à incuber les cellules sur un substrat de croissance,
(iv) procéder à une analyse pour détecter la formation d'une colonie de prions,
où la présence de colonies de prions indique la présence de prions dans l'échantillon.

2. Procédé selon la revendication 1, dans lequel l'incubation des cellules sur un substrat de croissance comprend les étapes consistant à :
(i) mettre à incuber les cellules jusqu'à ce qu'elles atteignent la confluence,
(ii) détacher les cellules du substrat de croissance,
(iii) mettre à incuber les cellules sur un substrat frais à une densité initiale d'approximativement un dixième de la densité de confluence.

3. Procédé selon la revendication 2, dans lequel les étapes (i) à (iii) de la revendication 2 sont exécutées deux fois supplémentaires.

4. Procédé selon l'une quelconque des revendications 1 à 3, dans lequel l'analyse pour rechercher la formation de colonies comprend les étapes consistant à :
(i) transférer les cellules vers un support,
(ii) visualiser là PrPSc,
(iii) noter la présence ou l'absence de colonies présentant ou montrant de la PrPSc.

5. Procédé selon l'une quelconque des revendications 1 à 4, dans lequel l'analyse pour rechercher la formation de colonies comprend les étapes consistant à :
(i) transférer les cellules sur un support de nitrocellulose,
(ii) dégrader la PrPc en traitant les cellules avec de la protéinase K,
(iii) visualiser la PrPSc par coloration avec un anticorps anti-PrP,
(iv) noter la présence ou l'absence de colonies présentant ou montrant de la PrPSc.

6. Procédé selon l'une quelconque des revendications 2 à 5, dans lequel le transfert des cellules sur un support comprend les étapes consistant à :
(i) détacher les cellules du substrat de croissance,
(ii) ensemencer les cellules sur des lamelles couvre-objet,
(iii) faire incuber les cellules pendant environ 4 jours,
(iv) déposer les cellules sur le support par effet buvard.

7. Procédé selon l'une quelconque des revendications 2 à 5, dans lequel le transfert des cellules sur un substrat comprend les étapes consistant à :
(i) détacher les cellules du substrat de croissance,
(ii) appliquer les cellules directement sur le support.

8. Procédé selon la revendication 7, dans lequel le support comprend une lame d'analyse ELISPOT.

9. Procédé selon l'une quelconque des revendications précédentes, dans lequel le substrat de croissance est une lamelle couvre-objet constituée de matière plastique ou de verre.

10. Procédé selon l'une quelconque des revendications précédentes, dans lequel l'incubation des cellules sur le substrat de croissance dure approximativement de 6 à 7 jours.

11. Procédé selon l'une quelconque des revendications précédentes, dans lequel les cellules sont mises en contact avec l'échantillon pendant approximativement 3 jours.

12. Procédé selon l'une quelconque des revendications précédentes, dans lequel l'échantillon comprend un matériau en phase solide.

13. Procédé selon la revendication 12, dans lequel les cellules sont mises en contact avec l'échantillon en permettant que les cellules adhèrent au matériau en phase solide et incubent pendant au moins trois jours.

14. Procédé selon la revendication 13, dans lequel les cellules sont mises à incuber avec le matériau en phase solide pendant approximativement 8 jours.

15. Procédé selon l'une quelconque des revendications 12 à 14, dans lequel le matériau en phase solide comprend le substrat de croissance.

16. Procédé selon l'une quelconque des revendications précédentes, dans lequel le prion qui est détecté est le RML.

17. Procédé de quantification de prions dans un échantillon comprenant l'exécution du procédé selon l'une quelconque des revendications précédentes, où l'étape consistant à effectuer une analyse pour rechercher la formation de colonies de prions comprend en outre l'étape consistant à compter le nombre de colonies.

18. Procédé selon la revendication 17, comprenant en outre l'estimation du nombre de prions dans l'échantillon en utilisant le nombre de colonies comptées pour déterminer le nombre de prions présents à partir d'une courbe d'étalonnage.

19. Procédé selon la revendication 18, dans lequel la courbe d'étalonnage est un tracé log-log.

20. Souche de cellules N2a/Gary déposée à l'ECACC sous le numéro d'accès 02031337 ou 03031801.
